# EUROPEAN PATENT SPECIFICATION

(11) **EP 4 125 724 B1**
(45) Date of publication and mention of the grant of the patent: **11.06.2025**
(21) Application number: 21719339.0
(22) Date of filing: 19.03.2021
(51) Int. Cl.: A61F 2/24, A61M 25/00

(54) **HIGH FLEXIBILITY IMPLANT CATHETER WITH LOW COMPRESSION**
HOCHFLEXIBLER IMPLANTATKATHETER MIT GERINGER KOMPRESSION
CATHÉTER D'IMPLANT À HAUTE FLEXIBILITÉ À FAIBLE COMPRESSION

(30) Priority: 31.03.2020 US 202063003125 P; 19.05.2020 US 202063027266 P
(43) Date of publication of application: 08.02.2023
(73) Proprietor: Edwards Lifesciences Corporation, Irvine, CA 92614 (US)
(72) Inventor: METCHIK, Asher, L., Irvine, CA 92614 (US); DIXON, Eric, Robert, Irvine, CA 92614 (US)
(74) Representative: Eisenführ Speiser
(86) International application number: PCT/US2021/023269
(87) International publication number: WO 2021/202130

(56) References cited:
- JP-A- 2012 139 345
- US-A1- 2019 008 639
- US-A1- 2019 314 604

## Description

### RELATED APPLICATION

The present application claims the benefit of U.S. Provisional Application No. 63/003,125, filed on March 31, 2020 and U.S. Provisional Application No. 63/027,266, filed on May 19, 2020.

### BACKGROUND

The native heart valves (i.e., the aortic, pulmonary, tricuspid, and mitral valves) serve critical functions in assuring the forward flow of an adequate supply of blood through the cardiovascular system. These heart valves can be damaged, and thus rendered less effective, for example, by congenital malformations, inflammatory processes, infectious conditions, disease, etc. Such damage to the valves can result in serious cardiovascular compromise or death. Damaged valves can be surgically repaired or replaced during open heart surgery. However, open heart surgeries are highly invasive, and complications may occur. Transvascular techniques can be used to introduce and implant prosthetic devices in a manner that is much less invasive than open heart surgery. As one example, a transvascular technique useable for accessing the native mitral and aortic valves is the trans-septal technique. The trans-septal technique comprises advancing a catheter into the right atrium (e.g., inserting a catheter into the right femoral vein, up the inferior vena cava and into the right atrium). The septum is then punctured, and the catheter passed into the left atrium. A similar transvascular technique can be used to implant a prosthetic device within the tricuspid valve that begins similarly to the trans-septal technique but stops short of puncturing the septum and instead turns the delivery catheter toward the tricuspid valve in the right atrium.

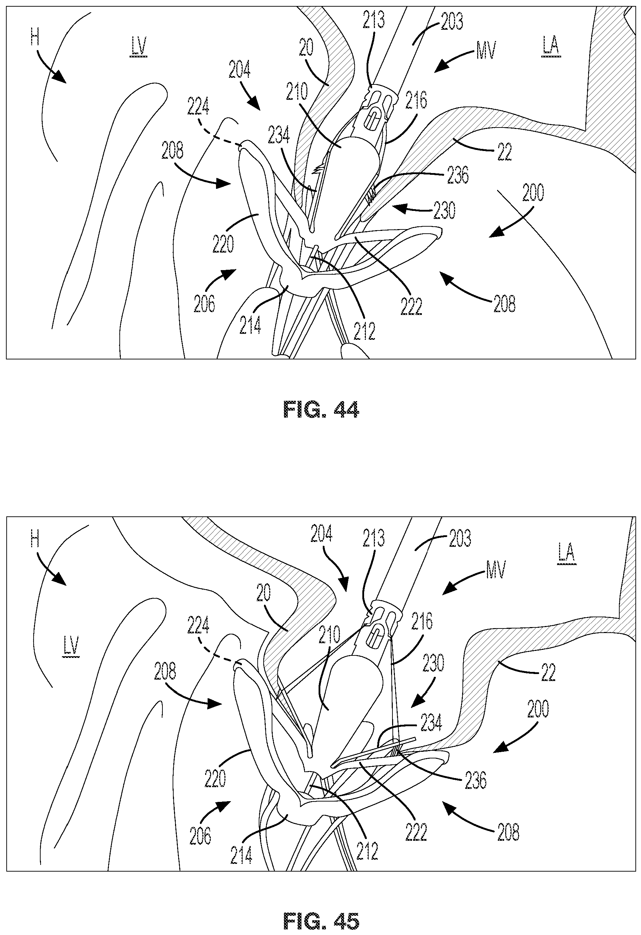

US 2019/0008639 A1 (US'639) discloses devices, systems and methods for delivering a prosthesis to a body location, for example for delivering a replacement mitral heart valve to a native mitral valve location (cf. abstract of US'639). Fig. 45B of US 639 illustrates a reinforced shaft for such a delivery system, the shaft having a reflow prevention layer 4505 located radially on top of an outer hypotube 104 and/or a reinforced inner layer 4506 (cf. [0240]-[0243] of US'639).

A healthy heart has a generally conical shape that tapers to a lower apex. The heart is four-chambered and comprises the left atrium, right atrium, left ventricle, and right ventricle. The left and right sides of the heart are separated by a wall generally referred to as the septum. The native mitral valve of the human heart connects the left atrium to the left ventricle. The mitral valve has a very different anatomy than other native heart valves. The mitral valve includes an annulus portion, which is an annular portion of the native valve tissue surrounding the mitral valve orifice, and a pair of cusps, or leaflets, extending downward from the annulus into the left ventricle. The mitral valve annulus can form a "D"-shaped, oval, or otherwise out-of-round cross-sectional shape having major and minor axes. The anterior leaflet can be larger than the posterior leaflet, forming a generally "C"-shaped boundary between the abutting sides of the leaflets when they are closed together.

When operating properly, the anterior leaflet and the posterior leaflet function together as a one-way valve to allow blood to flow only from the left atrium to the left ventricle. The left atrium receives oxygenated blood from the pulmonary veins. When the muscles of the left atrium contract and the left ventricle dilates (also referred to as "ventricular diastole" or "diastole"), the oxygenated blood that is collected in the left atrium flows into the left ventricle. When the muscles of the left atrium relax and the muscles of the left ventricle contract (also referred to as "ventricular systole" or "systole"), the increased blood pressure in the left ventricle urges the sides of the two leaflets together, thereby closing the one-way mitral valve so that blood cannot flow back to the left atrium and is instead expelled out of the left ventricle through the aortic valve. To prevent the two leaflets from prolapsing under pressure and folding back through the mitral annulus toward the left atrium, a plurality of fibrous cords called chordae tendineae tether the leaflets to papillary muscles in the left ventricle.

Valvular regurgitation involves the valve improperly allowing some blood to flow in the wrong direction through the valve. For example, mitral regurgitation occurs when the native mitral valve fails to close properly and blood flows into the left atrium from the left ventricle during the systolic phase of heart contraction. Mitral regurgitation is one of the most common forms of valvular heart disease. Mitral regurgitation can have many different causes, such as leaflet prolapse, dysfunctional papillary muscles, stretching of the mitral valve annulus resulting from dilation of the left ventricle, more than one of these, etc. Mitral regurgitation at a central portion of the leaflets can be referred to as central jet mitral regurgitation and mitral regurgitation nearer to one commissure (i.e., location where the leaflets meet) of the leaflets can be referred to as eccentric jet mitral regurgitation. Central jet regurgitation occurs when the edges of the leaflets do not meet in the middle and thus the valve does not close, and regurgitation is present. Tricuspid regurgitation can be similar, but on the right side of the heart.

### SUMMARY

The present invention relates to an implant catheter as defined in independent claim 1 and to a method for manufacturing an implant catheter as defined in independent claim 9. Preferred configurations of the invention are defined in dependent claims 2 to 8 and 10 to 15.

This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. Various features and steps as described elsewhere in this disclosure may be included in the examples summarized here.

In some implementations, an implant catheter comprises an outer jacket, one or more layers positioned inside the outer jacket, and a reinforcement layer positioned inside the outer jacket. At least one of the outer jacket and the one or more of the layers are reflowed to bond the jacket, the reinforcement layer, and the one or more layers together. A portion of the reinforcement layer is free from all material of the outer jacket and the one or more layers that are reflowed. The reinforcement layer can be a coil, coil layer, spring, spring layer, braid, braided layer, multiple of these, one or more combinations of these, etc.

In some implementations, an implant catheter comprises an outer jacket and one or more layers positioned inside the outer jacket. The implant catheter can also include a reinforcement layer positioned inside the outer jacket. The reinforcement layer can be a coil, coil layer, spring, spring layer, braid, braided layer, multiple of these, one or more combinations of these, etc.

In some implementations, at least one of the outer jacket and the one or more of the layers are reflowed to bond the jacket, the reinforcement layer (e.g., coil, coil layer, etc.), and the one or more layers together.

In some implementations, a portion of the reinforcement layer (e.g., coil layer, etc.) is free from all material of the outer jacket and the one or more layers.

In some implementations, the implant catheter further comprises a plurality of sub-lumens.

In some implementations, a leakage shield prevents material of the outer jacket and the one or more layers that are reflowed from contacting the portion of the reinforcement layer or coil layer. In some implementations, the reinforcement layer or coil layer is disposed inside the leakage shield.

In some implementations, a first portion of the outer jacket is formed from a material with an enhanced level of flexibility with regard to the remaining portions of the outer jacket.

In some implementations, the reinforcement layer (e.g., the coil layer, etc.) is formed from self-aligning wire.

In some implementations, an implant catheter comprises an outer jacket, a main lumen, and a plurality of layers positioned between the outer jacket and the main lumen. The implant catheter can include a reinforcement layer. The reinforcement layer can be a coil, coil layer, spring, spring layer, braid, braided layer, multiple of these, one or more combinations of these, etc.

In some implementations, a portion of at least one of the plurality of layers is omitted along a section of the implant catheter.

In some implementations, a leakage shield is located adjacent to the reinforcement layer or coil layer in the area of the omitted layer portion. In some implementations, the leakage shield is formed from polytetrafluoroethylene.

In some implementations, the implant catheter comprises a plurality of sub-lumens.

In some implementations, the reinforcement layer (e.g., a coil layer, etc.) comprises and/or is formed from self-aligning wire.

In some implementations, an implant catheter comprises an outer jacket, a main lumen, a plurality of layers positioned between the outer jacket and the main lumen, and a reinforcement layer. The reinforcement layer can be a coil, coil layer, spring, spring layer, braid, braided layer, multiple of these, one or more combinations of these, etc.

In some implementations, a first leakage shield is located adjacent to the reinforcement layer (e.g., coil layer, etc.) and situated between the reinforcement layer and the outer jacket.

In some implementations, a second leakage shield is located adjacent to the reinforcement layer and situated between the reinforcement layer and main lumen. In some implementations, the first and second leakage shield are formed from polytetrafluoroethylene.

In some implementations, the implant catheter comprises a plurality of sub-lumens.

In some implementations, a first portion of the outer jacket is formed from a material with an enhanced level of flexibility with regard to the remaining portions of the outer jacket.

In some implementations, the reinforcement layer is a coil layer formed from self-aligning wire.

In some implementations, a method of manufacturing an implant catheter comprises obtaining and/or providing an outer jacket and one or more additional layers. The method includes positioning the one or more additional layers inside the outer jacket. The method also includes positioning a reinforcement layer inside the outer jacket. The reinforcement layer can be a coil, coil layer, spring, spring layer, braid, braided layer, multiple of these, one or more combinations of these, etc.

In some implementations, the method includes reflowing at least one of the outer jacket and the one or more of the layers to bond the jacket, the reinforcement layer (e.g., coil, coil layer, etc.), and the one or more layers together.

In some implementations, a portion of the reinforcement layer is free from all material of the outer jacket and the one or more layers. In some implementations, the reinforcement layer is a coil layer, and the coil layer is free from all material of the outer jacket and the one or more layers.

In some implementations, the method includes forming one or more than one sub-lumens in the implant catheter. This can involve positioning wires and/or mandrels in the location(s) desired for the sub-lumen(s) prior to reflowing and/or can involve removing the wires and/or mandrels after reflowing.

In some implementations, the method includes using a leakage shield to prevent material of the outer jacket and the one or more layers from contacting the reinforcement layer during reflowing. In some implementations, the method includes positioning the reinforcement layer (e.g., the coil layer, etc.) inside the leakage shield.

In some implementations, obtaining includes obtaining the outer jacket, wherein a first portion of the outer jacket is formed from a material with an enhanced level of flexibility with regard to the remaining portions of the outer jacket.

In some implementations, the reinforcement layer (e.g., the coil layer, etc.) comprises and/or is formed from self-aligning wire.

In some implementations, a method of manufacturing an implant catheter comprises obtaining and/or providing an outer jacket and a plurality of layers. The method includes positioning the outer jacket and the plurality of layers such that the outer jacket is radially outside the plurality of layers and a main lumen is formed radially inside the outer jacket and the plurality of layers. The method also includes positioning a reinforcement layer inside the outer jacket and/or inside one or more of the plurality of layers. The reinforcement layer can be a coil, coil layer, spring, spring layer, braid, braided layer, multiple of these, one or more combinations of these, etc.

In some implementations, a portion of at least one of the plurality of layers is omitted along a section of the implant catheter. In some implementations, the method includes positioning a leakage shield adjacent to the reinforcement layer or coil layer in the area of the omitted layer portion. In some implementations, the leakage shield is formed from polytetrafluoroethylene.

In some implementations, the method includes reflowing at least one of the outer jacket and the plurality of layers to bond the jacket, the reinforcement layer (e.g., coil, coil layer, etc.), and the plurality of layers together.

In some implementations, the method includes forming one or more than one sub-lumens in the implant catheter. This can involve positioning wires and/or mandrels in the location(s) desired for the sub-lumen(s) prior to reflowing and/or can involve removing the wires and/or mandrels after reflowing.

In some implementations, the reinforcement layer (e.g., a coil layer, etc.) comprises and/or is formed from self-aligning wire.

In some implementations, a method of manufacturing an implant catheter comprises obtaining, positioning, and/or arranging an outer iacket a oluralitv of layers, and a reinforcement layer relative to each other such that the plurality of layers and the reinforcement layer are radially inside the outer jacket. A main lumen can be formed radially inside all of the outer jacket, the plurality of layers, and the reinforcement layer. The reinforcement layer can be a coil, coil layer, spring, spring layer, braid, braided layer, multiple of these, one or more combinations of these, etc.

In some implementations, the method includes arranging and/or positioning a first leakage shield adjacent to the reinforcement layer (e.g., coil layer, etc.) and situated between the reinforcement layer and the outer jacket.

In some implementations, the method includes arranging and/or positioning a second leakage shield adjacent to the reinforcement layer and situated radially inside the reinforcement layer and/or between the reinforcement layer and main lumen. In some implementations, the first and second leakage shield are formed from polytetrafluoroethylene.

In some implementations, the method includes reflowing at least one of the outer jacket and the plurality of layers to bond the jacket, the reinforcement layer (e.g., coil, coil layer, etc.), and the plurality of layers together.

In some implementations, the method includes forming one or more than one sub-lumens in the implant catheter. This can involve positioning wires and/or mandrels in the location(s) desired for the sub-lumen(s) prior to reflowing and/or can involve removing the wires and/or mandrels after reflowing.

In some implementations, a first portion of the outer jacket is formed from a material with an enhanced level of flexibility with regard to the remaining portions of the outer jacket.

In some implementations, the reinforcement layer is a coil layer formed from self-aligning wire.

A further understanding of the nature and advantages of the present invention are set forth in the following description and claims, particularly when considered in conjunction with the accompanying drawings in which like parts bear like reference numerals.

### BRIEF DESCRIPTION OF THE DRAWINGS

To further clarify various aspects of implementations of the present disclosure, a more particular description of the certain examples and implementations will be made by reference to various aspects of the appended drawings. It is appreciated that these drawings depict only example implementations of the present disclosure and are therefore not to be considered limiting of the scope of the disclosure. Moreover, while the figures can be drawn to scale for some examples, the figures are not necessarily drawn to scale for all examples. Examples and other features and advantages of the present disclosure will be described and explained with additional specificity and detail through the use of the accompanying drawings in which:
Figure 1 illustrates a cutaway view of the human heart in a diastolic phase;
Figure 2 illustrates a cutaway view of the human heart in a systolic phase;
Figure 3 illustrates a cutaway view of the human heart in a systolic phase showing mitral regurgitation;
Figure 4 is the cutaway view of Figure 3 annotated to illustrate a natural shape of mitral valve leaflets in the systolic phase;
Figure 5 illustrates a healthy mitral valve with the leaflets closed as viewed from an atrial side of the mitral valve;
Figure 6 illustrates a dysfunctional mitral valve with a visible gap between the leaflets as viewed from an atrial side of the mitral valve;
Figure 7 illustrates a tricuspid valve viewed from an atrial side of the tricuspid valve;
Figures 8-14 show an example of an implantable device or implant, in various stages of deployment;
Figure 15 shows an example of an implantable device or implant that is similar to the device illustrated by Figures 8-14, but where the paddles are independently controllable;
Figures 16-21 show the example implantable device or implant of Figures 8-14 being delivered and implanted within a native valve;
Figure 22 shows a perspective view of an example implantable device or implant in a closed position;
Figure 23 shows a front view of the implantable device or implant of Figure 22;
Figure 24 shows a side view of the implantable device or implant of Figure 22;
Figure 25 shows a front view of the implantable device or implant of Figure 22 with a cover covering the paddles and a coaptation element or spacer;
Figure 26 shows a top perspective view of the implantable device or implant of Figure 22 in an open position;
Figure 27 shows a bottom perspective view of the implantable device or implant of Figure 22 in an open position;
Figure 28 shows a clasp for use in an implantable device or implant;
Figure 29 shows a portion of native valve tissue grasped by a clasp;
Figure 30 shows a side view of an example implantable device or implant in a partially-open position with clasps in a closed position;
Figure 31 shows a side view of an example implantable device or implant in a partially-open position with clasps in an open position;
Figure 32 shows a side view of an example implantable device or implant in a half-open position with clasps in a closed position;
Figure 33 shows a side view of an example implantable device or implant in a half-open position with clasps in an open position;
Figure 34 shows a side view of an example implantable device or implant in a three-quarters-open position with clasps in a closed position;
Figure 35 shows a side view of an example implantable device or implant in a three-quarters-open position with clasps in an open position;
Figure 36 shows a side view of an example implantable device in a fully open or full bailout position with clasps in a closed position;
Figure 37 shows a side view of an example implantable device in a fully open or full bailout position with clasps in an open position;
Figures 38-49 show the example implantable device or implant of Figures 30-38, including a cover, being delivered and implanted within a native valve;
Figure 50 is a schematic view illustrating a path of native valve leaflets along each side of a coaptation element or spacer of an example valve repair device or implant;
Figure 51 is a top schematic view illustrating a path of native valve leaflets around a coaptation element or spacer of an example valve repair device or implant;
Figure 52 illustrates a coaptation element or spacer in a gap of a native valve as viewed from an atrial side of the native valve;
Figure 53 illustrates a valve repair device or implant attached to native valve leaflets with the coaptation element or spacer in the gap of the native valve as viewed from a ventricular side of the native valve;
Figure 54 is a perspective view of a valve repair device or implant attached to native valve leaflets with the coaptation element or spacer in the gap of the native valve shown from a ventricular side of the native valve;
Figure 55 shows a perspective view of an example implantable device or implant in a closed position;
Figure 56 shows a perspective view of an example clasp of an example implantable device or implant in a closed position;
Figure 57 illustrates a cross-section view of an implant catheter;
Figure 58 is a cross-section view of an implant catheter with a plurality of sub-lumens;
Figure 59 is a perspective view of an implant catheter with sub-lumens;
Figure 60 is a cross-section view of an example implant catheter;
Figure 61A is a cross-section view of an example implant catheter;
Figure 61B is a cross-section view of an example implant catheter of the invention;
Figure 62A is a cross-section view of an example coil for use in an implant catheter;
Figure 62B is a cross-section view of an example coil for use in an implant catheter;
Figures 63A and 63B are cross-section views of an example coil for using in an implant catheter, the coil wire having a rectangular cross section;
Figures 64A and 64B are cross-section views of an example coil for using in an implant catheter, the coil wire having a rectangular cross section; and
Figures 65A and 65B are cross-section views of an example flexible, self-aligning coil.

### DETAILED DESCRIPTION

The following description refers to the accompanying drawings, which illustrate example implementations of the present disclosure. Other implementations having different structures and operation do not depart from the scope of the present disclosure.

Example implementations of the present disclosure are directed to systems, devices, methods, etc. for repairing a defective heart valve. For example, various implementations of implantable devices, valve repair devices, implants, and sactuaystems (including systems for delivery thereof) are disclosed herein, and any combination of these options can be made unless specifically excluded. In other words, individual components of the disclosed devices and systems can be combined unless mutually exclusive or otherwise physically impossible. Further, the techniques and methods herein can be performed on a living animal or on a simulation, such as on a cadaver, cadaver heart, simulator (e.g. with the body parts, heart, tissue, etc. being simulated), etc.

As described herein, when one or more components are described as being connected, joined, affixed, coupled, attached, or otherwise interconnected, such interconnection can be direct as between the components or can be indirect such as through the use of one or more intermediary components. Also as described herein, reference to a "member," "component," or "portion" shall not be limited to a single structural member, component, or element but can include an assembly of components, members, or elements. Also as described herein, the terms "substantially" and "about" are defined as at least close to (and includes) a given value or state (preferably within 10% of, more preferably within 1% of, and most preferably within 0.1% of).

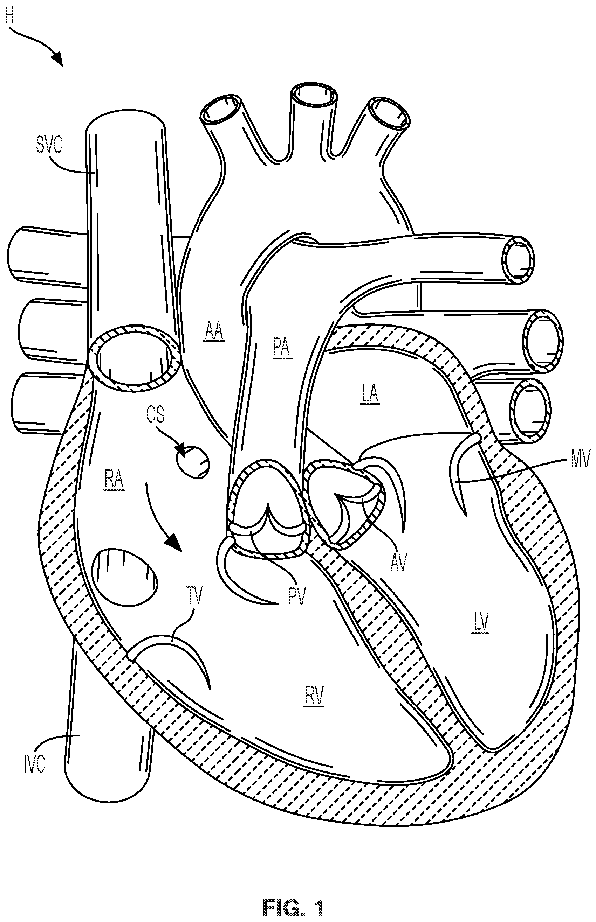

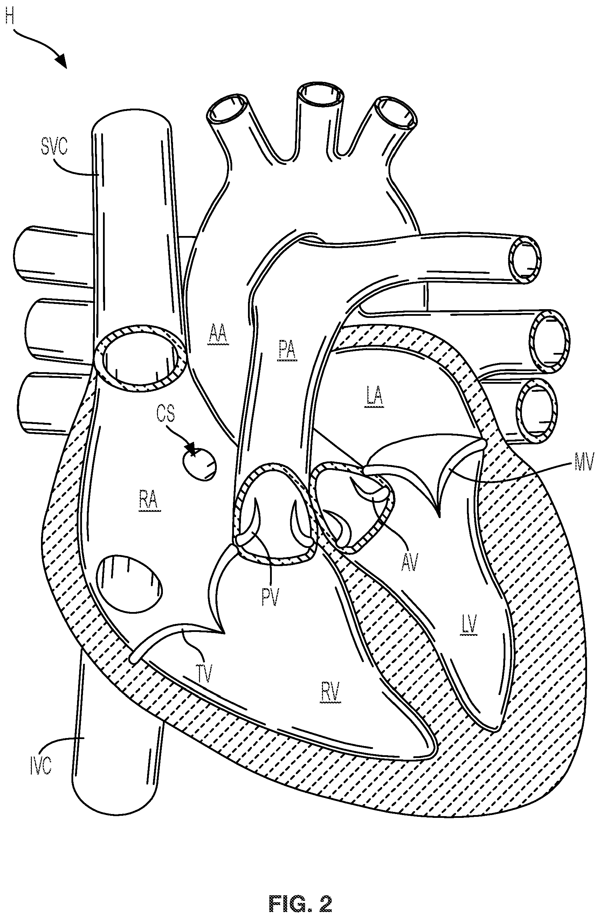

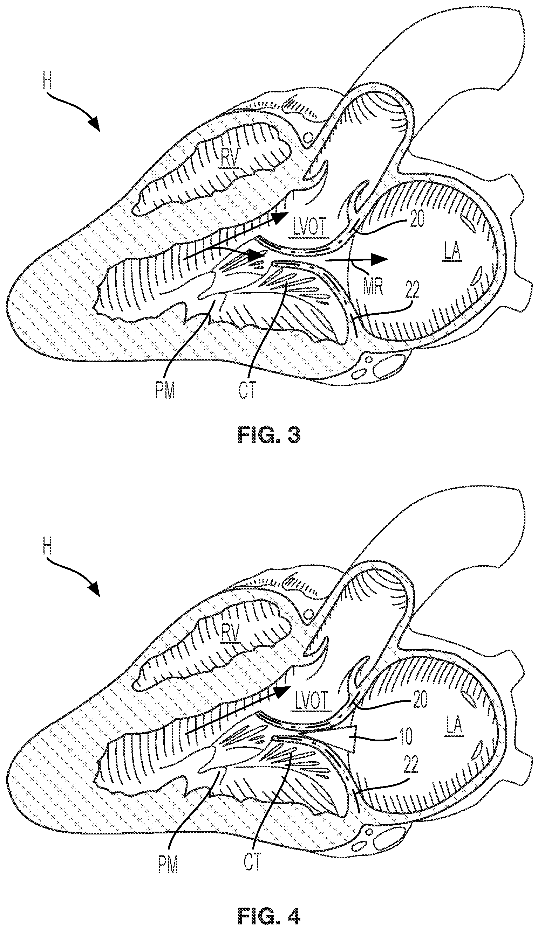

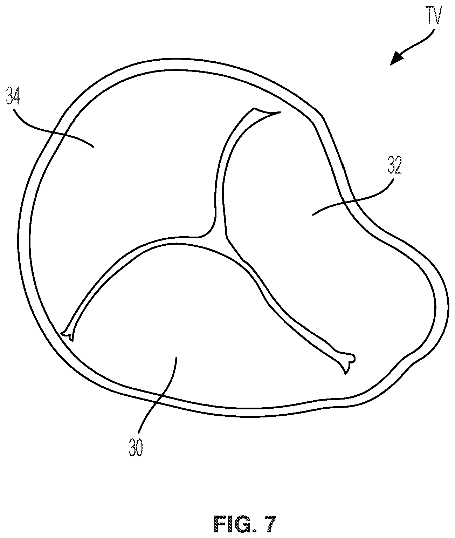

Figures 1 and 2 are cutaway views of the human heart H in diastolic and systolic phases, respectively. The right ventricle RV and left ventricle LV are separated from the right atrium RA and left atrium LA, respectively, by the tricuspid valve TV and mitral valve MV; i.e., the atrioventricular valves. Additionally, the aortic valve AV separates the left ventricle LV from the ascending aorta AA, and the pulmonary valve PV separates the right ventricle from the pulmonary artery PA. Each of these valves has flexible leaflets (e.g., leaflets 20, 22 shown in Figures 3-6 and leaflets 30, 32, 34 shown in Fig. 7) extending inward across the respective orifices that come together or "coapt" in the flow stream to form the one-way, fluid-occluding surfaces. The native valve repair systems of the present application are frequently described and/or illustrated with respect to the mitral valve MV. Therefore, anatomical structures of the left atrium LA and left ventricle LV will be explained in greater detail. However, the devices described herein can also be used in repairing other native valves, e.g., the devices can be used in repairing the tricuspid valve TV, the aortic valve AV, and the pulmonary valve PV.

The left atrium LA receives oxygenated blood from the lungs. During the diastolic phase, or diastole, seen in Figure 1, the blood that was previously collected in the left atrium LA (during the systolic phase) moves through the mitral valve MV and into the left ventricle LV by expansion of the left ventricle LV. In the systolic phase, or systole, seen in Figure 2, the left ventricle LV contracts to force the blood through the aortic valve AV and ascending aorta AA into the body. During systole, the leaflets of the mitral valve MV close to prevent the blood from regurgitating from the left ventricle LV and back into the left atrium LA and blood is collected in the left atrium from the pulmonary vein. In some implementations, the devices described by the present application are used to repair the function of a defective mitral valve MV. That is, the devices are configured to help close the leaflets of the mitral valve to prevent blood from regurgitating from the left ventricle LV and back into the left atrium LA. Many of the devices described in the present application are designed to easily grasp and secure the native leaflets around a coaptation element or spacer that beneficially acts as a filler in the regurgitant orifice to prevent or inhibit back flow or regurgitation during systole, though this is not necessary.

Referring now to Figures 1-7, the mitral valve MV includes two leaflets, the anterior leaflet 20 and the posterior leaflet 22. The mitral valve MV also includes an annulus 24, which is a variably dense fibrous ring of tissues that encircles the leaflets 20, 22. Referring to Figures 3 and 4, the mitral valve MV is anchored to the wall of the left ventricle LV by chordae tendineae CT. The chordae tendineae CT are cord-like tendons that connect the papillary muscles PM (i.e., the muscles located at the base of the chordae tendineae CT and within the walls of the left ventricle LV) to the leaflets 20, 22 of the mitral valve MV. The papillary muscles PM serve to limit the movements of leaflets 20, 22 of the mitral valve MV and prevent the mitral valve MV from being reverted. The mitral valve MV opens and closes in response to pressure changes in the left atrium LA and the left ventricle LV. The papillary muscles PM do not open or close the mitral valve MV. Rather, the papillary muscles PM support or brace the leaflets 20, 22 against the high pressure needed to circulate blood throughout the body. Together the papillary muscles PM and the chordae tendineae CT are known as the subvalvular apparatus, which functions to keep the mitral valve MV from prolapsing into the left atrium LA when the mitral valve closes. As seen from a Left Ventricular Outflow Tract (LVOT) view shown in Figure 3, the anatomy of the leaflets 20, 22 is such that the inner sides of the leaflets coapt at the free end portions and the leaflets 20, 22 start receding or spreading apart from each other. The leaflets 20, 22 spread apart in the atrial direction, until each leaflet meets with the mitral annulus.

Various disease processes can impair proper function of one or more of the native valves of the heart H. These disease processes include degenerative processes (e.g., Barlow's Disease, fibroelastic deficiency, etc.), inflammatory processes (e.g., Rheumatic Heart Disease), and infectious processes (e.g., endocarditis, etc.). In addition, damage to the left ventricle LV or the right ventricle RV from prior heart attacks (i.e., myocardial infarction secondary to coronary artery disease) or other heart diseases (e.g., cardiomyopathy, etc.) can distort a native valve's geometry, which can cause the native valve to dysfunction. However, the majority of patients undergoing valve surgery, such as surgery to the mitral valve MV, suffer from a degenerative disease that causes a malfunction in a leaflet (e.g., leaflets 20, 22) of a native valve (e.g., the mitral valve MV), which results in prolapse and regurgitation.

Generally, a native valve may malfunction in different ways: including (1) valve stenosis; and (2) valve regurgitation. Valve stenosis occurs when a native valve does not open completely and thereby causes an obstruction of blood flow. Typically, valve stenosis results from buildup of calcified material on the leaflets of a valve, which causes the leaflets to thicken and impairs the ability of the valve to fully open to permit forward blood flow. Valve regurgitation occurs when the leaflets of the valve do not close completely thereby causing blood to leak back into the prior chamber (e.g., causing blood to leak from the left ventricle to the left atrium).

There are three main mechanisms by which a native valve becomes regurgitant-or incompetent-which include Carpentier's type I, type II, and type III malfunctions. A Carpentier type I malfunction involves the dilation of the annulus such that normally functioning leaflets are distracted from each other and fail to form a tight seal (i.e., the leaflets do not coapt properly). Included in a type I mechanism malfunction are perforations of the leaflets, as are present in endocarditis. A Carpentier's type II malfunction involves prolapse of one or more leaflets of a native valve above a plane of coaptation. A Carpentier's type III malfunction involves restriction of the motion of one or more leaflets of a native valve such that the leaflets are abnormally constrained below the plane of the annulus. Leaflet restriction can be caused by rheumatic disease (Ma) or dilation of a ventricle (IIIb).

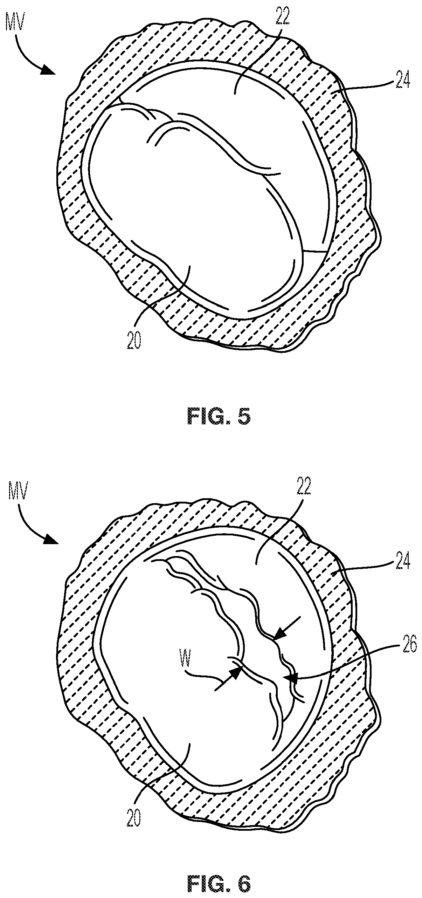

Referring to Figure 5, when a healthy mitral valve MV is in a closed position, the anterior leaflet 20 and the posterior leaflet 22 coapt, which prevents blood from leaking from the left ventricle LV to the left atrium LA. Referring to Figures 3 and 6, mitral regurgitation MR occurs when the anterior leaflet 20 and/or the posterior leaflet 22 of the mitral valve MV is displaced into the left atrium LA during systole so that the edges of the leaflets 20, 22 are not in contact with each other. This failure to coapt causes a gap 26 between the anterior leaflet 20 and the posterior leaflet 22, which allows blood to flow back into the left atrium LA from the left ventricle LV during systole, as illustrated by the mitral regurgitation MR flow path shown in Figure 3. Referring to Figure 6, the gap 26 can have a width W between about 2.5 mm and about 17.5 mm, between about 5 mm and about 15 mm, between about 7.5 mm and about 12.5 mm, or about 10 mm. In some situations, the gap 26 can have a width W greater than 15 mm. As set forth above, there are several different ways that a leaflet (e.g. leaflets 20, 22 of mitral valve MV) may malfunction which can thereby lead to valvular regurgitation.

In any of the above-mentioned situations, a valve repair device or implant is desired that is capable of engaging the anterior leaflet 20 and the posterior leaflet 22 to close the gap 26 and prevent regurgitation of blood through the mitral valve MV. As can be seen in Figure 4, an abstract representation of an implantable device, valve repair device, or implant 10 is shown implanted between the leaflets 20, 22 such that regurgitation does not occur during systole (compare Figure 3 with Figure 4). In some implementations, the coaptation element (e.g., spacer, coaption element, gap filler, etc.) of the device 10 has a generally tapered or triangular shape that naturally adapts to the native valve geometry and to its expanding leaflet nature (toward the annulus). In this application, the terms spacer, coaption element, coaptation element, and gap filler are used interchangeably and refer to an element that fills a portion of the space between native valve leaflets and/or that is configured such that the native valve leaflets engage or "coapt" against (e.g., such that the native leaflets coapt against the coaption element, coaptation element, spacer, etc. instead of only against one another).).

Although stenosis or regurgitation can affect any valve, stenosis is predominantly found to affect either the aortic valve AV or the pulmonary valve PV, and regurgitation is predominantly found to affect either the mitral valve MV or the tricuspid valve TV. Both valve stenosis and valve regurgitation increase the workload of the heart H and may lead to very serious conditions if left un-treated; such as endocarditis, congestive heart failure, permanent heart damage, cardiac arrest, and ultimately death. Because the left side of the heart (i.e., the left atrium LA, the left ventricle LV, the mitral valve MV, and the aortic valve AV) are primarily responsible for circulating the flow of blood throughout the body. Accordingly, because of the substantially higher pressures on the left side heart dysfunction of the mitral valve MV or the aortic valve AV is particularly problematic and often life threatening.

Malfunctioning native heart valves may either be repaired or replaced. Repair typically involves the preservation and correction of the patient's native valve. Replacement typically involves replacing the patient's native valve with a biological or mechanical substitute. Typically, the aortic valve AV and pulmonary valve PV are more prone to stenosis. Because stenotic damage sustained by the leaflets is irreversible, treatments for a stenotic aortic valve or stenotic pulmonary valve can be removal and replacement of the valve with a surgically implanted heart valve, or displacement of the valve with a transcatheter heart valve. The mitral valve MV and the tricuspid valve TV are more prone to deformation of leaflets and/or surrounding tissue, which, as described above, prevents the mitral valve MV or tricuspid valve TV from closing properly and allows for regurgitation or back flow of blood from the ventricle into the atrium (e.g., a deformed mitral valve MV may allow for regurgitation or back flow from the left ventricle LV to the left atrium LA as shown in Figure 3). The regurgitation or back flow of blood from the ventricle to the atrium results in valvular insufficiency. Deformations in the structure or shape of the mitral valve MV or the tricuspid valve TV are often repairable. In addition, regurgitation can occur due to the chordae tendineae CT becoming dysfunctional (e.g., the chordae tendineae CT may stretch or rupture), which allows the anterior leaflet 20 and the posterior leaflet 22 to be reverted such that blood is regurgitated into the left atrium LA. The problems occurring due to dysfunctional chordae tendineae CT can be repaired by repairing the chordae tendineae CT or the structure of the mitral valve MV (e.g., by securing the leaflets 20, 22 at the affected portion of the mitral valve).

The devices and procedures disclosed herein often make reference to repairing the structure of a mitral valve. However, it should be understood that the devices and concepts provided herein can be used to repair any native valve, as well as any component of a native valve. Such devices can be used between the leaflets 20, 22 of the mitral valve MV to prevent or inhibit regurgitation of blood from the left ventricle into the left atrium. With respect to the tricuspid valve TV (Figure 7), any of the devices and concepts herein can be used between any two of the anterior leaflet 30, septal leaflet 32, and posterior leaflet 34 to prevent or inhibit regurgitation of blood from the right ventricle into the right atrium. In addition, any of the devices and concepts provided herein can be used on all three of the leaflets 30, 32, 34 together to prevent or inhibit regurgitation of blood from the right ventricle to the right atrium. That is, the valve repair devices or implants provided herein can be centrally located between the three leaflets 30, 32, 34.

An example implantable device (e.g., implantable prosthetic device, etc.) or implant can optionally have a coaptation element (e.g., spacer, coaption element, gap filler, etc.) and at least one anchor (e.g., one, two, three, or more). In some implementations, an implantable device or implant can have any combination or sub-combination of the features disclosed herein without a coaptation element. When included, the coaptation element (e.g., coaption element, spacer, etc.) is configured to be positioned within the native heart valve orifice to help fill the space between the leaflets and form a more effective seal, thereby reducing or preventing regurgitation described above. The coaptation element can have a structure that is impervious to blood (or that resists blood flow therethrough) and that allows the native leaflets to close around the coaptation element during ventricular systole to block blood from flowing from the left or right ventricle back into the left or right atrium, respectively. The device or implant can be configured to seal against two or three native valve leaflets; that is, the device may be used in the native mitral (bicuspid) and tricuspid valves. The coaptation element is sometimes referred to herein as a spacer because the coaptation element can fill a space between improperly functioning native leaflets (e.g., mitral leaflets 20, 22 or tricuspid leaflets 30, 32, 34) that do not close completely.

The optional coaptation element (e.g., spacer, coaption element, etc.) can have various shapes. In some implementations, the coaptation element can have an elongated cylindrical shape having a round cross-sectional shape. In some implementations, the coaptation element can have an oval cross-sectional shape, an ovoid cross-sectional shape, a crescent cross-sectional shape, a rectangular cross-sectional shape, or various other non-cylindrical shapes. In some implementations, the coaptation element can have an atrial portion positioned in or adjacent to the atrium, a ventricular or lower portion positioned in or adjacent to the ventricle, and a side surface that extends between the native leaflets. In some implementations configured for use in the tricuspid valve, the atrial or upper portion is positioned in or adjacent to the right atrium, and the ventricular or lower portion is positioned in or adjacent to the right ventricle, and the side surface that extends between the native tricuspid leaflets.

In some implementations, the anchor can be configured to secure the device to one or both of the native leaflets such that the coaptation element is positioned between the two native leaflets. In some implementations configured for use in the tricuspid valve, the anchor is configured to secure the device to one, two, or three of the tricuspid leaflets such that the coaptation element is positioned between the three native leaflets. In some implementations, the anchor can attach to the coaptation element at a location adjacent the ventricular portion of the coaptation element. In some implementations, the anchor can attach to an actuation element, such as a shaft or actuation wire, to which the coaptation element is also attached. In some implementations, the anchor and the coaptation element can be positioned independently with respect to each other by separately moving each of the anchor and the coaptation element along the longitudinal axis of the actuation element (e.g., actuation shaft, actuation rod, actuation tube, actuation wire, etc.). In some implementations, the anchor and the coaptation element can be positioned simultaneously by moving the anchor and the coaptation element together along the longitudinal axis of the actuation element, e.g., shaft, actuation wire, etc.). The anchor can be configured to be positioned behind a native leaflet when implanted such that the leaflet is grasped by the anchor.

The device or implant can be configured to be implanted via a delivery system or other means for delivery. The delivery system can comprise one or more of a guide/delivery sheath, a delivery catheter, a steerable catheter, an implant catheter, tube, combinations of these, etc. The coaptation element and the anchor can be compressible to a radially compressed state and can be self-expandable to a radially expanded state when compressive pressure is released. The device can be configured for the anchor to be expanded radially away from the still-compressed coaptation element initially in order to create a gap between the coaptation element and the anchor. A native leaflet can then be positioned in the gap. The coaptation element can be expanded radially, closing the gap between the coaptation element and the anchor and capturing the leaflet between the coaptation element and the anchor. In some implementations, the anchor and coaptation element are optionally configured to self-expand. The implantation methods for various implementations can be different and are more fully discussed below with respect to each implementation. Additional information regarding these and other delivery methods can be found in U.S. Pat. No. 8,449,599 and U.S. Patent Application Publication Nos. 2014/0222136, 2014/0067052, 2016/0331523, and PCT patent application publication Nos. WO2020/076898. These method(s) can be performed on a living animal or on a simulation, such as on a cadaver, cadaver heart, simulator (e.g. with the body parts, heart, tissue, etc. being simulated), etc. *mutatis mutandis.*

The disclosed devices or implants can be configured such that the anchor is connected to a leaflet, taking advantage of the tension from native chordae tendineae to resist high systolic pressure urging the device toward the left atrium. During diastole, the devices can rely on the compressive and retention forces exerted on the leaflet that is grasped by the anchor.

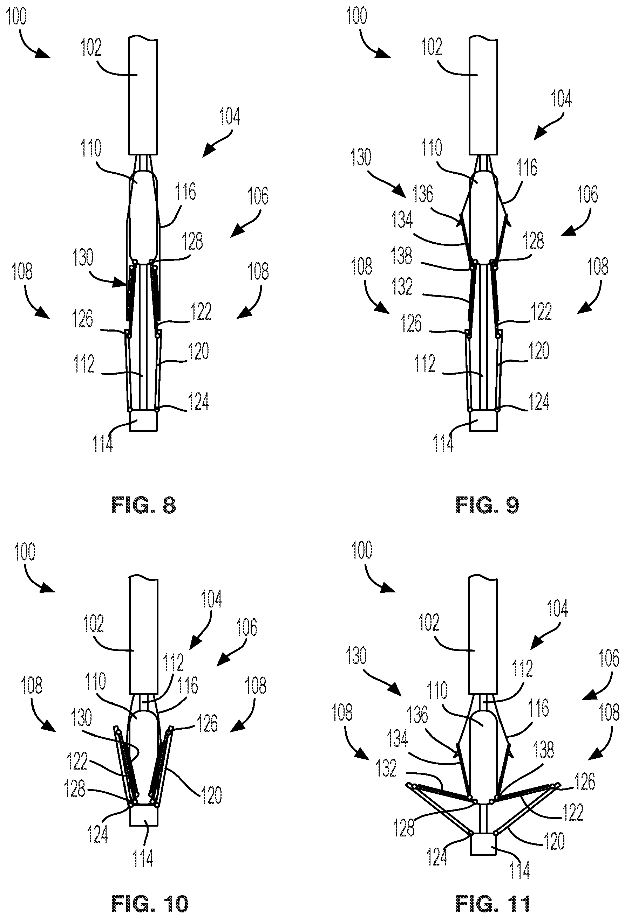

Referring now to Figures 8-15, a schematically illustrated implantable device or implant 100 (e.g., a prosthetic spacer device, valve repair device, etc.) is shown in various stages of deployment. The device or implant 100 and other similar devices/implants are described in more detail in PCT patent application publication Nos. WO2018/195215, WO2020/076898, and WO 2019/139904. The device 100 can include any other features for an implantable device or implant discussed in the present application or the applications cited above, and the device 100 can be positioned to engage valve tissue (e.g., leaflets 20, 22, 30, 32, 34) as part of any suitable valve repair system (e.g., any valve repair system disclosed in the present application or the applications cited above).

The device or implant 100 is deployed from a delivery system or other means for delivery 102. The delivery system 102 can comprise one or more of a catheter, a sheath, a guide catheter/sheath, a delivery catheter/sheath, a steerable catheter, an implant catheter, a tube, a channel, a pathway, combinations of these, etc. The device or implant 100 includes a coaption or coaptation portion 104 and an anchor portion 106.

In some implementations, the coaptation portion 104 of the device or implant 100 includes a coaptation element or means for coapting 110 (e.g., spacer, plug, filler, foam, sheet, membrane, coaption element, etc.) that is adapted to be implanted between leaflets of a native valve (e.g., a native mitral valve, native tricuspid valve, etc.) and is slidably attached to an actuation element 112 (e.g., actuation wire, actuation shaft, actuation tube, etc.). The anchor portion 106 includes one or more anchors 108 that are actuatable between open and closed conditions and can take a wide variety of forms, such as, for example, paddles, gripping elements, or the like. Actuation of the means for actuating or actuation element 112 opens and closes the anchor portion 106 of the device 100 to grasp the native valve leaflets during implantation. The means for actuating or actuation element 112 (as well as other means for actuating and actuation elements herein) can take a wide variety of different forms (e.g., as a wire, rod, shaft, tube, screw, suture, line, strip, combination of these, etc.), be made of a variety of different materials, and have a variety of configurations. As one example, the actuation element can be threaded such that rotation of the actuation element moves the anchor portion 106 relative to the coaptation portion 104. Or, the actuation element can be unthreaded, such that pushing or pulling the actuation element 112 moves the anchor portion 106 relative to the coaptation portion 104.

The anchor portion 106 and/or anchors of the device 100 include outer paddles 120 and inner paddles 122 that are, in some implementations, connected between a cap 114 and the means for coapting or coaptation element 110 by portions 124, 126, 128. The portions 124, 126, 128 can be jointed and/or flexible to move between all of the positions described below. The interconnection of the outer paddles 120, the inner paddles 122, the coaptation element 110, and the cap 114 by the portions 124, 126, and 128 can constrain the device to the positions and movements illustrated herein.

In some implementations, the delivery system 102 includes a steerable catheter, implant catheter, and means for actuating or actuation element 112 (e.g., actuation wire, actuation shaft, etc.). These can be configured to extend through a guide catheter/sheath (e.g., a transseptal sheath, etc.). In some implementations, the means for actuating or actuation element 112 extends through a delivery catheter and the means for coapting or coaptation element 110 to the distal end (e.g., a cap 114 or other attachment portion at the distal connection of the anchor portion 106). Extending and retracting the actuation element 112 increases and decreases the spacing between the coaptation element 110 and the distal end of the device (e.g., the cap 114 or other attachment portion), respectively. In some implementations, a collar or other attachment element removably attaches the coaptation element 110 to the delivery system 102, either directly or indirectly, so that the means for actuating or actuation element 112 slides through the collar or other attachment element and, in some implementations, through a means for coapting or coaptation element 110 during actuation to open and close the paddles 120, 122 of the anchor portion 106 and/or anchors 108.

In some implementation, the anchor portion 106 and/or anchors 108 can include attachment portions or gripping members. The illustrated gripping members can comprise clasps 130 that include a base or fixed arm 132, a moveable arm 134, optional barbs, friction-enhancing elements, or other means for securing 136 (e.g., protrusions, ridges, grooves, textured surfaces, adhesive, etc.), and a joint portion 138. The fixed arms 132 are attached to the inner paddles 122. In some implementations, the fixed arms 132 are attached to the inner paddles 122 with the joint portion 138 disposed proximate means for coapting or coaptation element 110. In some implementations, the clasps (e.g., barbed clasps, etc.) have flat surfaces and do not fit in a recess of the inner paddle. Rather, the flat portions of the clasps are disposed against the surface of the inner paddle 122. The joint portion 138 provides a spring force between the fixed and moveable arms 132, 134 of the clasp 130. The joint portion 138 can be any suitable joint, such as a flexible joint, a spring joint, a pivot joint, or the like. In some implementations, the joint portion 138 is a flexible piece of material integrally formed with the fixed and moveable arms 132, 134. The fixed arms 132 are attached to the inner paddles 122 and remain stationary or substantially stationary relative to the inner paddles 122 when the moveable arms 134 are opened to open the clasps 130 and expose the barbs, friction-enhancing elements, or means for securing 136.

In some implementations, the clasps 130 are opened by applying tension to actuation lines 116 attached to the moveable arms 134, thereby causing the moveable arms 134 to articulate, flex, or pivot on the joint portions 138. The actuation lines 116 extend through the delivery system 102 (e.g., through a steerable catheter and/or an implant catheter). Other actuation mechanisms are also possible.

The actuation line 116 can take a wide variety of forms, such as, for example, a line, a suture, a wire, a rod, a catheter, or the like. The clasps 130 can be spring loaded so that in the closed position the clasps 130 continue to provide a pinching force on the grasped native leaflet. This pinching force remains constant regardless of the position of the inner paddles 122. Optional barbs, friction-enhancing elements, or other means for securing 136 of the clasps 130 can grab, pinch, and/or pierce the native leaflets to further secure the native leaflets.

During implantation, the paddles 120, 122 can be opened and closed, for example, to grasp the native leaflets (e.g., native mitral valve leaflets, etc.) between the paddles 120, 122 and/or between the paddles 120, 122 and a means for coapting or coaptation element 110. The clasps 130 can be used to grasp and/or further secure the native leaflets by engaging the leaflets with barbs, friction-enhancing elements, or means for securing 136 and pinching the leaflets between the moveable and fixed arms 134, 132. The barbs, friction-enhancing elements, or other means for securing 136 (e.g., barbs, protrusions, ridges, grooves, textured surfaces, adhesive, etc.) of the clasps or barbed clasps 130 increase friction with the leaflets or may partially or completely puncture the leaflets. The actuation lines 116 can be actuated separately so that each clasp 130 can be opened and closed separately. Separate operation allows one leaflet to be grasped at a time, or for the repositioning of a clasp 130 on a leaflet that was insufficiently grasped, without altering a successful grasp on the other leaflet. The clasps 130 can be opened and closed relative to the position of the inner paddle 122 (as long as the inner paddle is in an open or at least partially open position), thereby allowing leaflets to be grasped in a variety of positions as the particular situation requires.

Referring now to Figure 8, the device 100 is shown in an elongated or fully open condition for deployment from an implant delivery catheter of the delivery system 102. The device 100 is disposed at the end of the catheter 102 in the fully open position, because the fully open position takes up the least space and allows the smallest catheter to be used (or the largest device 100 to be used for a given catheter size). In the elongated condition the cap 114 is spaced apart from the means for coapting or coaptation element 110 such that the paddles 120, 122 are fully extended. In some implementations, an angle formed between the interior of the outer and inner paddles 120, 122 is approximately 180 degrees. The clasps 130 are kept in a closed condition during deployment through the delivery system 102 so that the barbs, friction-enhancing elements, or other means for securing 136 (Figure 9) do not catch or damage the delivery system 102 or tissue in the patient's heart. The actuation lines 116 can extend through the coupler 117, around the collar 115, and attach to the moveable arms 134.

Referring now to Figure 9, the device 100 is shown in an elongated detangling condition, similar to Figure 8, but with the clasps 130 in a fully open position, ranging from about 140 degrees to about 200 degrees, from about 170 degrees to about 190 degrees, or about 180 degrees between fixed and moveable portions 132, 134 of the clasps 130. Fully opening the paddles 120, 122 and the clasps 130 has been found to improve ease of detanglement or detachment from anatomy of the patient, such as the chordae tendineae CT, during implantation of the device 100.

Referring now to Figure 10, the device 100 is shown in a shortened or fully closed condition. The compact size of the device 100 in the shortened condition allows for easier maneuvering and placement within the heart. To move the device 100 from the elongated condition to the shortened condition, the means for actuating or actuation element 112 is retracted to pull the cap 114 towards the means for coapting or coaptation element 110. The connection portion(s) 126 (e.g., joint(s), flexible connection(s), etc.) between the outer paddle 120 and inner paddle 122 are constrained in movement such that compression forces acting on the outer paddle 120 from the cap 114 being retracted towards the means for coapting or coaptation element 110 cause the paddles or gripping elements to move radially outward. During movement from the open to closed position, the outer paddles 120 maintain an acute angle with the means for actuating or actuation element 112. The outer paddles 120 can optionally be biased toward a closed position. The inner paddles 122 during the same motion move through a considerably larger angle as they are oriented away from the means for coapting or coaptation element 110 in the open condition and collapse along the sides of the means for coapting or coaptation element 110 in the closed condition. In some implementations, the inner paddles 122 are thinner and/or narrower than the outer paddles 120, and the connection portions 126, 128 (e.g., joints, flexible connections, etc.) connected to the inner paddles 122 can be thinner and/or more flexible. For example, this increased flexibility can allow more movement than the connection portion 124 connecting the outer paddle 120 to the cap 114. In some implementations, the outer paddles 120 are narrower than the inner paddles 122. The connection portions 126, 128 connected to the inner paddles 122 can be more flexible, for example, to allow more movement than the connection portion 124 connecting the outer paddle 120 to the cap 114. In some implementations, the inner paddles 122 can be the same or substantially the same width as the outer paddles

Referring now to Figures 11-13, the device 100 is shown in a partially open, grasp-ready condition. To transition from the fully closed to the partially open condition, the means for actuating or actuation element (e.g., actuation wire, actuation shaft, etc.) is extended to push the cap 114 away from the means for coapting or coaptation element 110, thereby pulling on the outer paddles 120, which in turn pull on the inner paddles 122, causing the anchors or anchor portion 106 to partially unfold. The actuation lines 116 are also retracted to open the clasps 130 so that the leaflets can be grasped. In some implementations, the pair of inner and outer paddles 122, 120 are moved in unison, rather than independently, by a single means for actuating or single actuation element 112. Also, the positions of the clasps 130 are dependent on the positions of the paddles 122, 120. For example, referring to Figure 10 closing the paddles 122, 120 also closes the clasps. In some implementations, the paddles 120, 122 can be independently controllable. For example, the device 100 can have two actuation elements and two independent caps (or other attachment portions), such that one independent actuation element (e.g., wire, shaft, etc.) and cap (or other attachment portion) are used to control one paddle, and the other independent actuation element and cap (or other attachment portion) are used to control the other paddle.

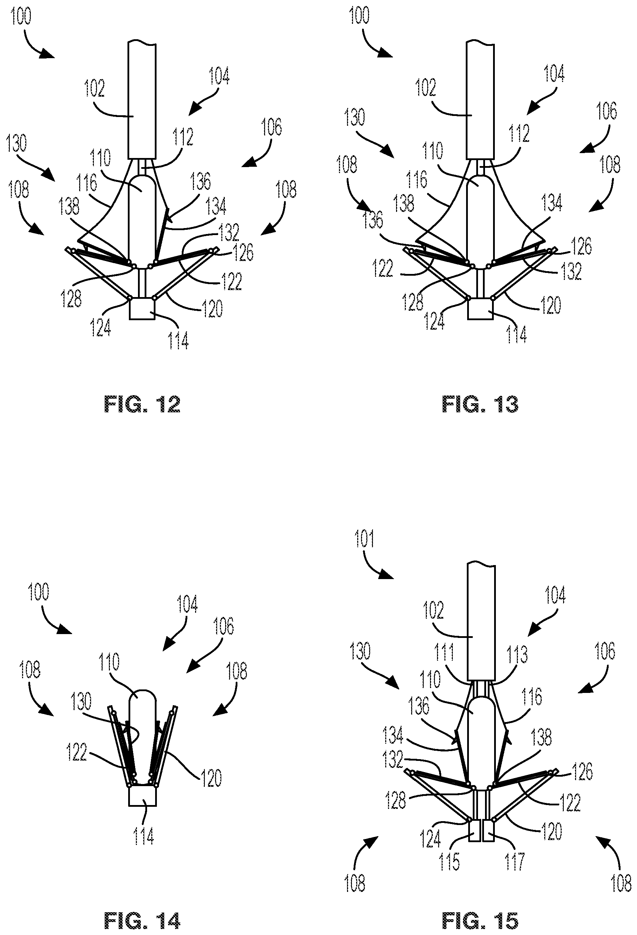

Referring now to Figure 12, one of the actuation lines 116 is extended to allow one of the clasps 130 to close. Referring now to Figure 13, the other actuation line 116 is extended to allow the other clasp 130 to close. Either or both of the actuation lines 116 can be repeatedly actuated to repeatedly open and close the clasps 130.

Referring now to Figure 14, the device 100 is shown in a fully closed and deployed condition. The delivery system or means for delivery 102 and means for actuating or actuation element 112 are retracted and the paddles 120, 122 and clasps 130 remain in a fully closed position. Once deployed, the device 100 can be maintained in the fully closed position with a mechanical latch or can be biased to remain closed through the use of spring materials, such as steel, other metals, plastics, composites, etc. or shape-memory alloys such as Nitinol. For example, the connection portions 124, 126, 128, the joint portions 138, and/or the inner and outer paddles 122, and/or an additional biasing component (not shown) can be formed of metals such as steel or shape-memory alloy, such as Nitinol-produced in a wire, sheet, tubing, or laser sintered powder-and are biased to hold the outer paddles 120 closed around the means for coapting or coaptation element 110 and the clasps 130 pinched around native leaflets. Similarly, the fixed and moveable arms 132, 134 of the clasps 130 are biased to pinch the leaflets. In some implementations, the attachment or connection portions 124, 126, 128, joint portions 138, and/or the inner and outer paddles 122, and/or an additional biasing component (not shown) can be formed of any other suitably elastic material, such as a metal or polymer material, to maintain the device 100 in the closed condition after implantation.

Figure 15 illustrates an example where the paddles 120, 122 are independently controllable. The device 100 illustrated by Figure 15 is similar to the device illustrated by Figure 11, except the device 100 of Figure 15 includes an actuation element that is configured as two independent actuation elements or actuation wires 111, 113 that are coupled to two independent caps 115, 117. To transition a first inner paddle 122 and a first outer paddle 120 from the fully closed to the partially open condition, the means for actuating or actuation element 111 is extended to push the cap 115 away from the means for coapting or coaptation element 110, thereby pulling on the outer paddle 120, which in turn pulls on the inner paddle 122, causing the first anchor 108 to partially unfold. To transition a second inner paddle 122 and a second outer paddle 120 from the fully closed to the partially open condition, the means for actuating or actuation element 113 is extended to push the cap 115 away from the means for coapting or coaptation element 110, thereby pulling on the outer paddle 120, which in turn pulls on the inner paddle 122, causing the second anchor 108 to partially unfold. The independent paddle control illustrated by Figure 15 can be implemented on any of the devices disclosed by the present application. For comparison, in the example illustrated by Figure 11, the pair of inner and outer paddles 122, 120 are moved in unison, rather than independently, by a single means for actuating or actuation element 112.

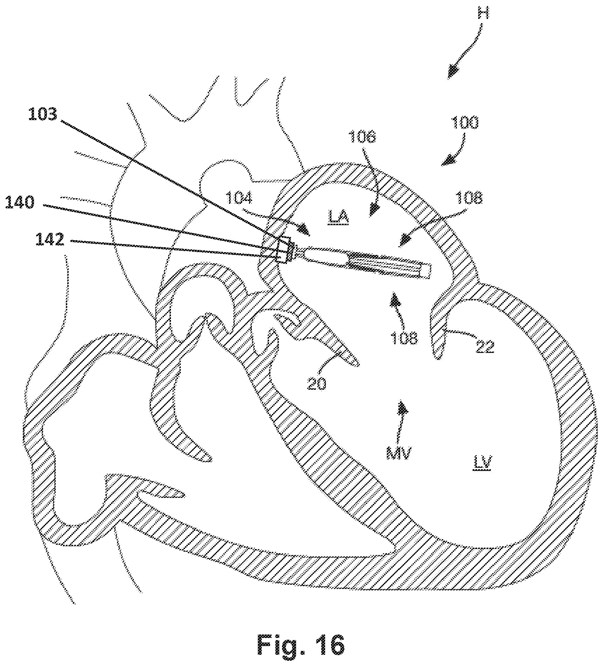

Referring now to Figures 16-21, the implantable device 100 of Figures 8-14 is shown being delivered and implanted within the native mitral valve MV of the heart H. Referring to Figure 16, a delivery sheath/catheter 142 is inserted into the left atrium LA through the septum. The delivery sheath/catheter 142 contains a steerable catheter 140, the implant catheter 103, and the implant/device 100. In one example implementation, the implant/device 100 is adjacent to an end of the steerable catheter 140 inside the delivery sheath/catheter 142, with the implant catheter 103 inside the steerable catheter 140. The delivery sheath/catheter 142 and/or the implant catheter 103 can optionally be steerable. In one example implementation, the delivery sheath/catheter 142 and/or the steerable catheter 140 are steerable, but the implant catheter 103 is passive or not steerable (except due to following the path of the steerable catheter).

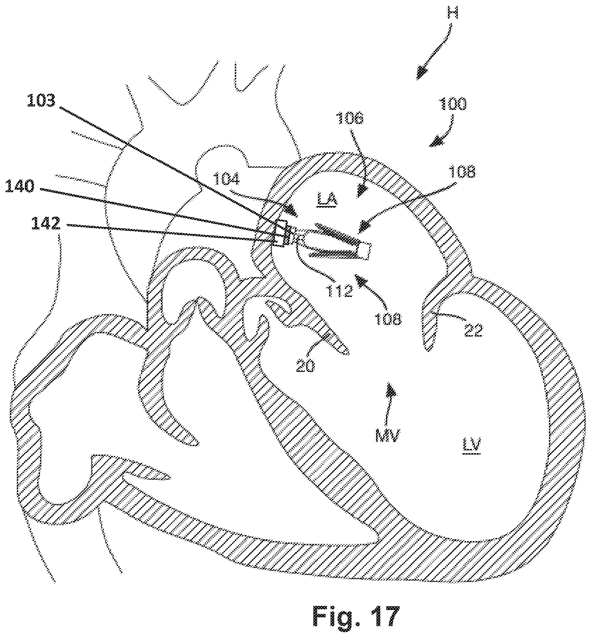

Once the delivery sheath/catheter 142 is in the left atrium, the implant/device 100 is deployed from the delivery catheter/sheath 142 in the fully open condition as illustrated in Figure 16. The means for actuating or actuation element 112 is then retracted to move the implant/device 100 into the fully closed condition shown in Figure 17.

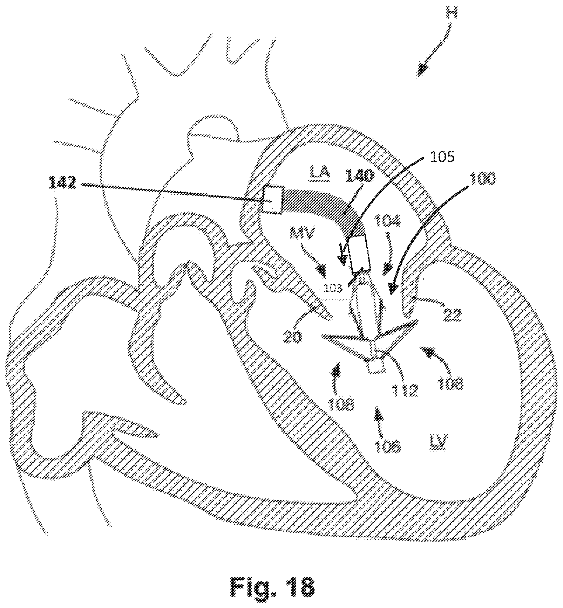

As can be seen in Figure 18, the implant/device 100 is moved into position within the mitral valve MV into the ventricle LV and partially opened so that the leaflets 20, 22 can be grasped. For example, the steerable catheter 140 can be advanced and steered or flexed to position the steerable catheter as illustrated by Figure 18. The implant catheter 102 connected to the implant/device 100 can be advanced (as indicated by arrow 105) from inside the steerable catheter 140 to position the implant as illustrated by Figure 18.

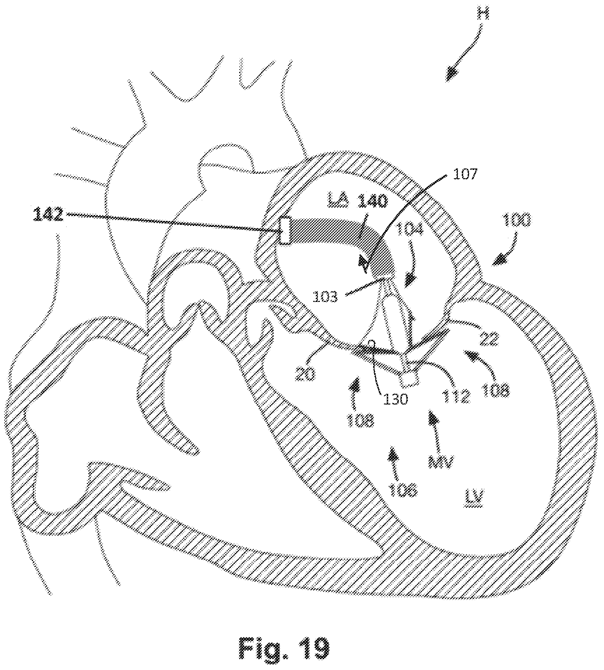

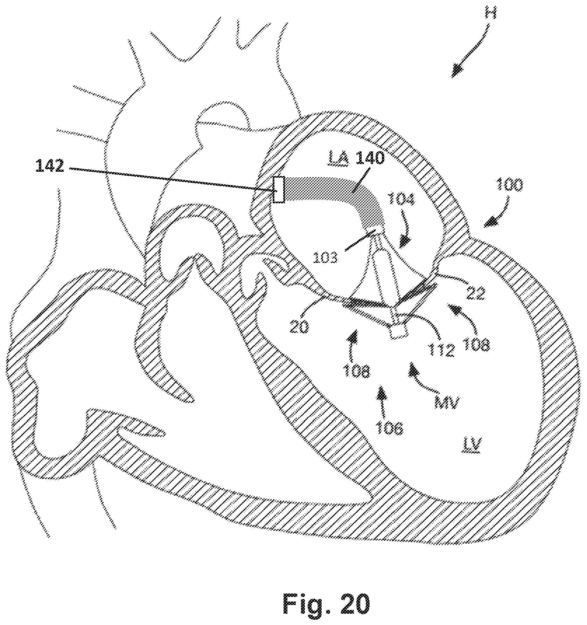

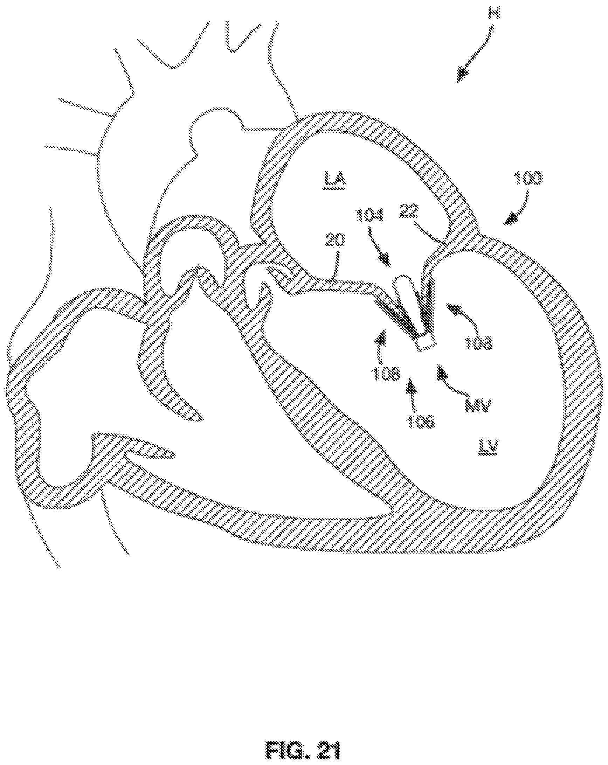

Referring now to Figure 19, the implant catheter 103 can be retracted into the steerable catheter 140 (as indicated by arrow 107) to position the mitral valve leaflets 20, 22 in the clasps 130 of the implant/device 100. An actuation line 116 is extended to close one of the clasps 130, capturing a leaflet 20. Figure 20 shows the other actuation line 116 being then extended to close the other clasp 130, capturing the remaining leaflet 22. Lastly, as can be seen in Figure 21, the delivery system 102 (e.g., steerable catheter 140, implant catheter 103, etc.), means for actuating or actuation element 112 and actuation lines 116 are then retracted and the implant/device 100 is fully closed and deployed in the native mitral valve MV.

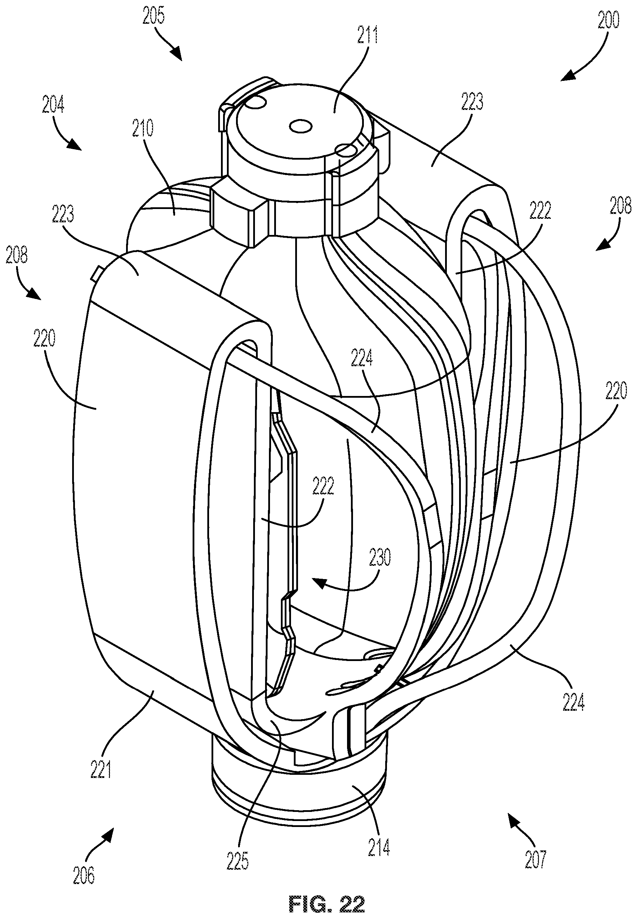

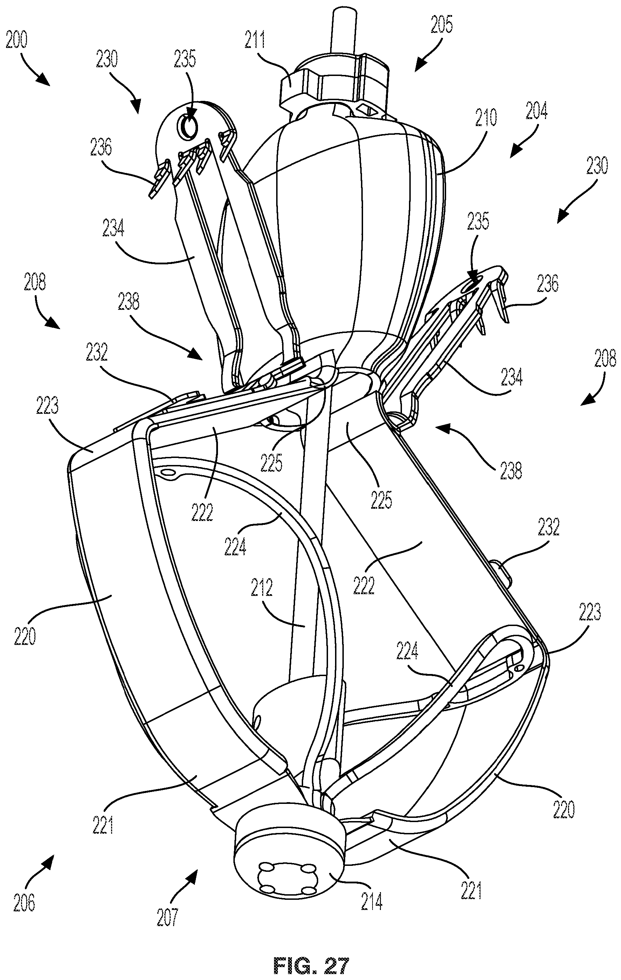

Referring now to Figures 22-27, an example of an implantable device or implant 200 is shown. The implantable device 200 is one of the many different configurations that the device 100 that is schematically illustrated in Figures 8-14 can take. The device 200 can include any other features for an implantable device or implant discussed in the present application, and the device 200 can be positioned to engage valve tissue 20, 22 as part of any suitable valve repair system (e.g., any valve repair system disclosed in the present application). The device/implant 200 can be a prosthetic spacer device, valve repair device, or another type of implant that attaches to leaflets of a native valve.

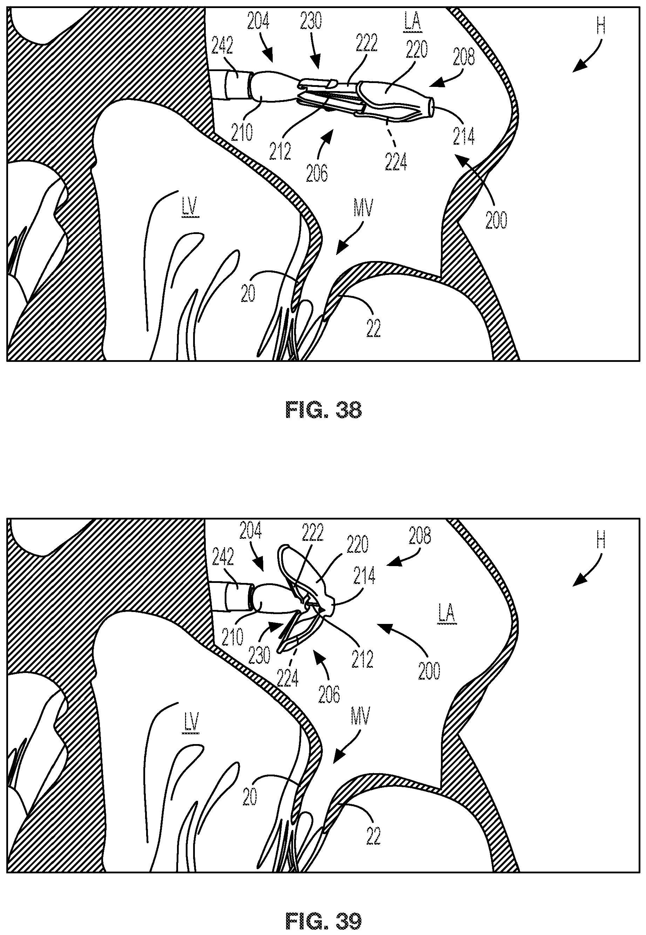

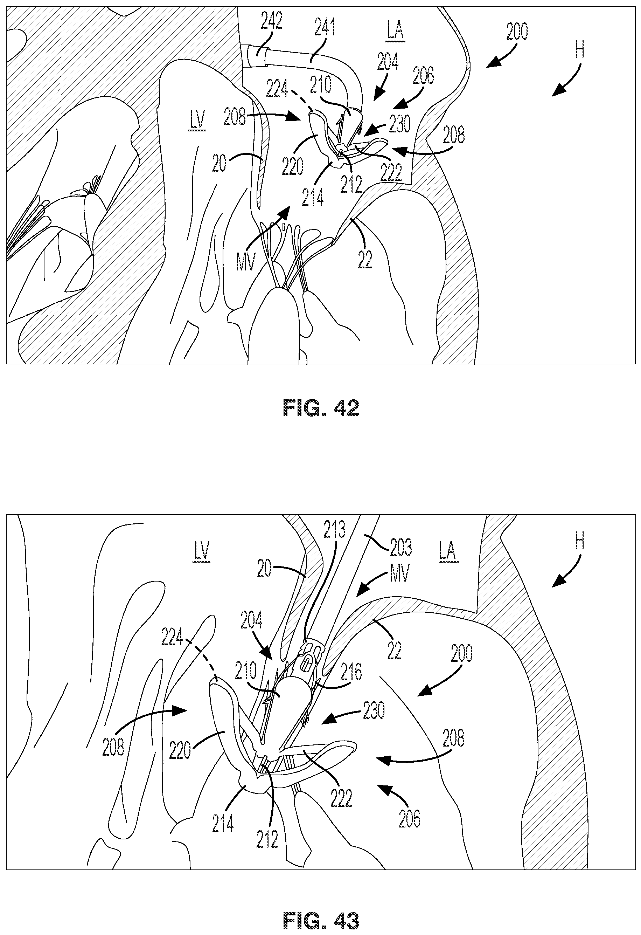

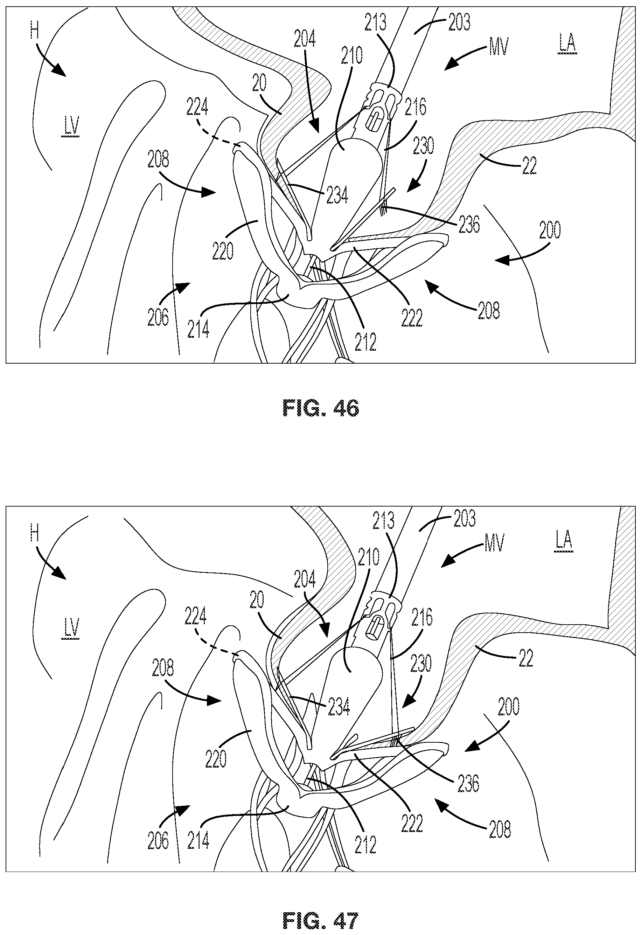

In some implementations, the implantable device or implant 200 includes a coaption or coaptation portion 204, a proximal or attachment portion 205, an anchor portion 206, and a distal portion 207. In some implementations, the coaption or coaptation portion 204 of the device optionally includes a coaptation element 210 (e.g., a spacer, coaption element, plug, membrane, sheet, etc.) for implantation between leaflets of a native valve. In some implementations, the anchor portion 206 includes a plurality of anchors 208. The anchors can be configured in a variety of ways. In some implementations, each anchor 208 includes outer paddles 220, inner paddles 222, paddle extension members or paddle frames 224, and clasps 230. In some implementations, the attachment portion 205 includes a first or proximal collar 211 (or other attachment element) for engaging with a capture mechanism 213 (Figures 43-49) of a delivery system (Figures 38-42 and 49). The delivery system can be the same as or similar to delivery system 102 described elsewhere and can comprise one or more of a catheter, a sheath, a guide catheter/sheath, a delivery catheter/sheath, a steerable catheter, an implant catheter, a tube, a channel, a pathway, combinations of these, etc.

In some implementations, the coaptation element 210 and paddles 220, 222 are formed from a flexible material that can be a metal fabric, such as a mesh, woven, braided, or formed in any other suitable way or a laser cut or otherwise cut flexible material. The material can be cloth, shape-memory alloy wire-such as Nitinol-to provide shape-setting capability, or any other flexible material suitable for implantation in the human body.

An actuation element 212 (e.g., actuation shaft, actuation rod, actuation tube, actuation wire, actuation line, etc.) extends from an implant catheter 241 (Figures 38-42) of the delivery system to engage and enable actuation of the implantable device or implant 200. In some implementations, the actuation element 212 extends through the capture mechanism 213, proximal collar 211, and coaptation element 210 to engage a cap 214 of the distal portion 207. The actuation element 212 can be configured to removably engage the cap 214 with a threaded connection, or the like, so that the actuation element 212 can be disengaged and removed from the device 200 after implantation.

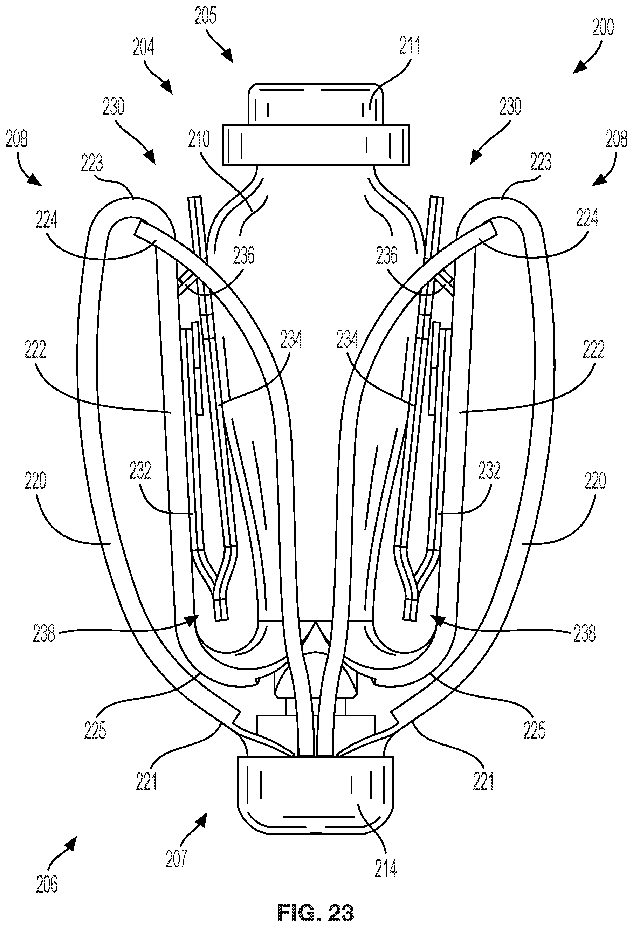

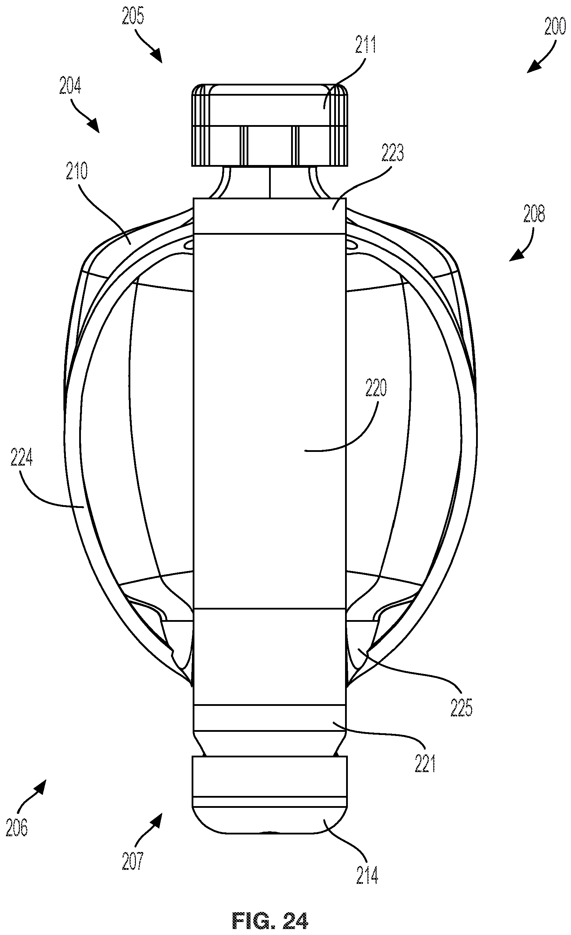

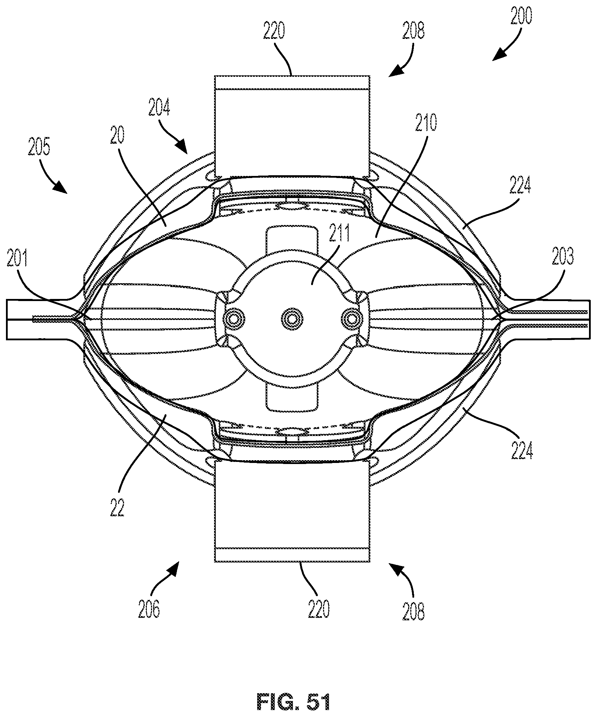

The coaptation element 210 extends from the proximal collar 211 (or other attachment element) to the inner paddles 222. In some implementations, the coaptation element 210 has a generally elongated and round shape, though other shapes and configurations are possible. In some implementations, the coaptation element 210 has an elliptical shape or cross-section when viewed from above (e.g., Figure 51) and has a tapered shape or cross-section when seen from a front view (e.g., Figure 23) and a round shape or cross-section when seen from a side view (e.g., Figure 24). A blend of these three geometries can result in the three-dimensional shape of the illustrated coaptation element 210 that achieves the benefits described herein. The round shape of the coaptation element 210 can also be seen, when viewed from above, to substantially follow or be close to the shape of the paddle frames 224.

The size and/or shape of the coaptation element 210 can be selected to minimize the number of implants that a single patient will require (preferably one), while at the same time maintaining low transvalvular gradients. In some implementations, the anterior-posterior distance at the top of the coaptation element is about 5 mm, and the medial-lateral distance of the coaptation element at its widest is about 10 mm. In some implementations, the overall geometry of the device 200 can be based on these two dimensions and the overall shape strategy described above. It should be readily apparent that the use of other anterior-posterior distance anterior-posterior distance and medial-lateral distance as starting points for the device will result in a device having different dimensions. Further, using other dimensions and the shape strategy described above will also result in a device having different dimensions.

In some implementations, the outer paddles 220 are jointably attached to the cap 214 of the distal portion 207 by connection portions 221 and to the inner paddles 222 by connection portions 223. The inner paddles 222 are jointably attached to the coaptation element by connection portions 225. In this manner, the anchors 208 are configured similar to legs in that the inner paddles 222 are like upper portions of the legs, the outer paddles 220 are like lower portions of the legs, and the connection portions 223 are like knee portions of the legs.

In some implementations, the inner paddles 222 are stiff, relatively stiff, rigid, have rigid portions and/or are stiffened by a stiffening member or a fixed portion 232 of the clasps 230. The stiffening of the inner paddle allows the device to move to the various different positions shown and described herein. The inner paddle 222, the outer paddle 220, the coaptation can all be interconnected as described herein, such that the device 200 is constrained to the movements and positions shown and described herein.

In some implementations, the paddle frames 224 are attached to the cap 214 at the distal portion 207 and extend to the connection portions 223 between the inner and outer paddles 222, 220. In some implementations, the paddle frames 224 are formed of a material that is more rigid and stiff than the material forming the paddles 222, 220 so that the paddle frames 224 provide support for the paddles 222, 220.

The paddle frames 224 provide additional pinching force between the inner paddles 222 and the coaptation element 210 and assist in wrapping the leaflets around the sides of the coaptation element 210 for a better seal between the coaptation element 210 and the leaflets, as can be seen in Figure 51. That is, the paddle frames 224 can be configured with a round three-dimensional shape extending from the cap 214 to the connection portions 223 of the anchors 208. The connections between the paddle frames 224, the outer and inner paddles 220, 222, the cap 214, and the coaptation element 210 can constrain each of these parts to the movements and positions described herein. In particular the connection portion 223 is constrained by its connection between the outer and inner paddles 220, 222 and by its connection to the paddle frame 224. Similarly, the paddle frame 224 is constrained by its attachment to the connection portion 223 (and thus the inner and outer paddles 222, 220) and to the cap 214.

Configuring the paddle frames 224 in this manner provides increased surface area compared to the outer paddles 220 alone. This can, for example, make it easier to grasp and secure the native leaflets. The increased surface area can also distribute the clamping force of the paddles 220 and paddle frames 224 against the native leaflets over a relatively larger surface of the native leaflets in order to further protect the native leaflet tissue. Referring again to Figure 51, the increased surface area of the paddle frames 224 can also allow the native leaflets to be clamped to the implantable device or implant 200, such that the native leaflets coapt entirely around the coaptation member or coaptation element 210. This can, for example, improve sealing of the native leaflets 20, 22 and thus prevent or further reduce mitral regurgitation.

In some implementations the clasps comprise a moveable arm coupled to the anchors. In some implementations, the clasps 230 include a base or fixed arm 232, a moveable arm 234, barbs 236, and a joint portion 238. The fixed arms 232 are attached to the inner paddles 222, with the joint portion 238 disposed proximate the coaptation element 210. The joint portion 238 is spring-loaded so that the fixed and moveable arms 232, 234 are biased toward each other when the clasp 230 is in a closed condition. In some implementations, the clasps 230 include friction-enhancing elements or means for securing, such as barbs, protrusions, ridges, grooves, textured surfaces, adhesive, etc.

In some implementations, the fixed arms 232 are attached to the inner paddles 222 through holes or slots 231 with sutures (not shown). The fixed arms 232 can be attached to the inner paddles 222 with any suitable means, such as screws or other fasteners, crimped sleeves, mechanical latches or snaps, welding, adhesive, clamps, latches, or the like. The fixed arms 232 remain substantially stationary relative to the inner paddles 222 when the moveable arms 234 are opened to open the clasps 230 and expose the barbs or other friction-enhancing elements 236. The clasps 230 are opened by applying tension to actuation lines 216 (e.g., as shown in Figures 43-48) attached to holes 235 in the moveable arms 234, thereby causing the moveable arms 234 to articulate, pivot, and/or flex on the joint portions 238.

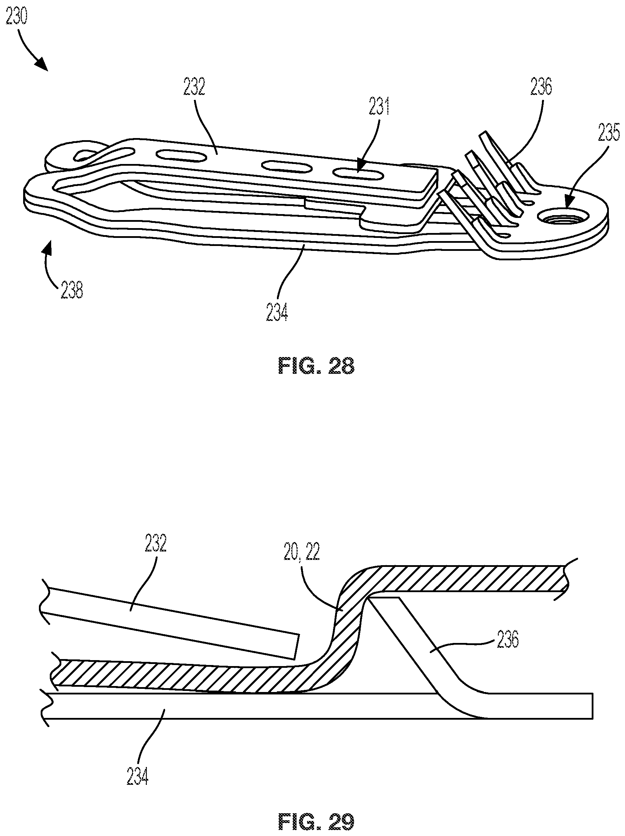

Referring now to Figure 29, a close-up view of one of the leaflets 20, 22 grasped by a clasp such as clasp 230 is shown. The leaflet 20, 22 is grasped between the moveable and fixed arms 234 of the clasp 230. The tissue of the leaflet 20, 22 is not pierced by the barbs or friction-enhancing elements 236, though in some implementations the barbs 236 may partially or fully pierce through the leaflet 20, 22. The angle and height of the barbs or friction-enhancing elements 236 relative to the moveable arm 234 helps to secure the leaflet 20, 22 within the clasp 230. In particular, a force pulling the implant off of the native leaflet 20, 22 will encourage the barbs or friction-enhancing elements 236 to further engage the tissue, thereby ensuring better retention. Retention of the leaflet 20, 22 in the clasp 230 is further improved by the position of fixed arm 232 near the barbs/friction-enhancing elements 236 when the clasp 230 is closed. In this arrangement, the tissue is formed by the fixed arms 232 and the moveable arms 234 and the barbs/friction-enhancing elements 236 into an S-shaped torturous path. Thus, forces pulling the leaflet 20, 22 away from the clasp 230 will encourage the tissue to further engage the barbs/friction-enhancing elements 236 before the leaflets 20, 22 can escape. For example, leaflet tension during diastole can encourage the barbs 236 to pull toward the end portion of the leaflet 20, 22. Thus, the S-shaped path can utilize the leaflet tension during diastole to more tightly engage the leaflets 20, 22 with the barbs/friction-enhancing elements 236.

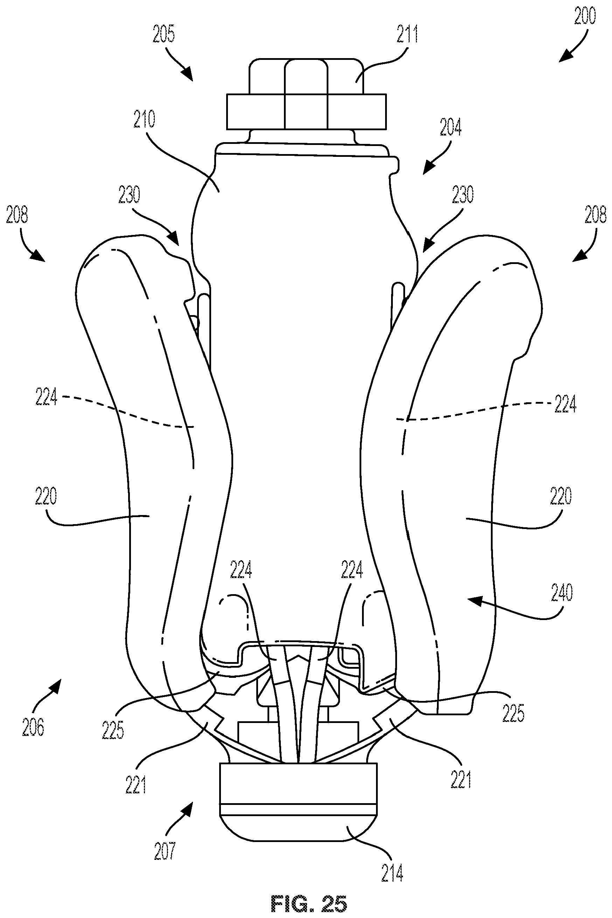

Referring to Figure 25, the prosthetic device or implant 200 can also include a cover 240. In some implementations, the cover 240 can be disposed on the coaptation element 210, the outer and inner paddles 220, 222, and/or the paddle frames 224. The cover 240 can be configured to prevent or reduce blood-flow through the prosthetic device or implant 200 and/or to promote native tissue ingrowth. In some implementations, the cover 240 can be a cloth or fabric such as PET, velour, or other suitable fabric. In some implementations, in lieu of or in addition to a fabric, the cover 240 can include a coating (e.g., polymeric) that is applied to the implantable prosthetic device or implant 200.

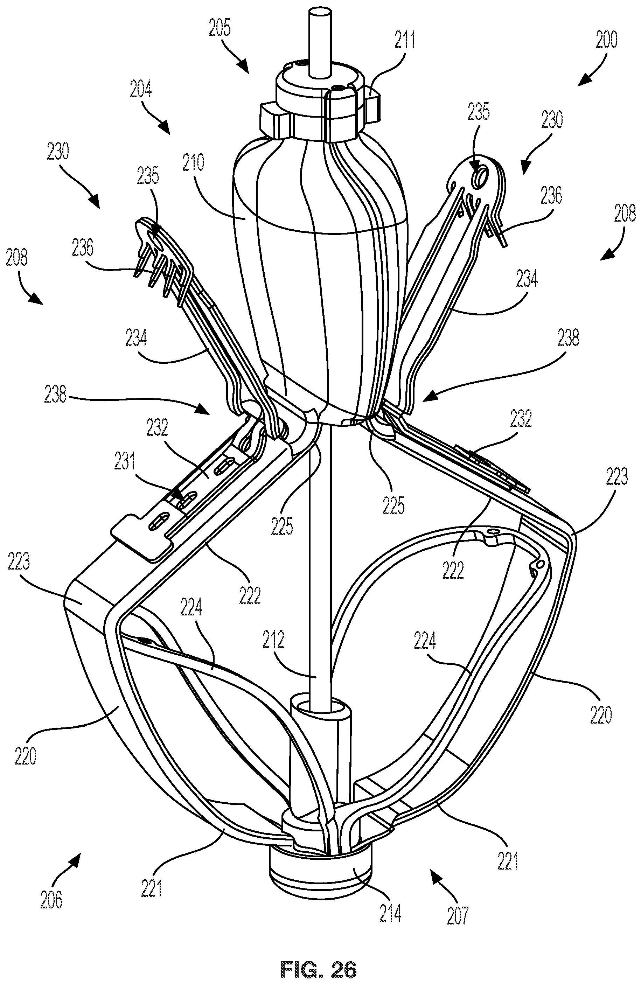

During implantation, the paddles 220, 222 of the anchors 208 are opened and closed to grasp the native valve leaflets 20, 22 between the paddles 220, 222 and the coaptation element 210. The anchors 208 are moved between a closed position (Figures 22-25) to various open positions (Figures 26-37) by extending and retracting the actuation element 212. Extending and retracting the actuation element 212 increases and decreases the spacing between the coaptation element 210 and the cap 214, respectively. The proximal collar 211 (or other attachment element) and the coaptation element 210 slide along the actuation element 212 during actuation so that changing of the spacing between the coaptation element 210 and the cap 214 causes the paddles 220, 220 to move between different positions to grasp the native valve leaflets 20, 22 during implantation.

As the device 200 is opened and closed, the pair of inner and outer paddles 222, 220 are moved in unison, rather than independently, by a single actuation element 212. Also, the positions of the clasps 230 are dependent on the positions of the paddles 222, 220. For example, the clasps 230 are arranged such that closure of the anchors 208 simultaneously closes the clasps 230. In some implementations, the device 200 can be made to have the paddles 220, 222 be independently controllable in the same manner (e.g., the device 100 illustrated in Figure 15).

In some implementations, the clasps 230 further secure the native leaflets 20, 22 by engaging the leaflets 20, 22 with barbs and/or other friction-enhancing elements 236 and pinching the leaflets 20, 22 between the moveable and fixed arms 234, 232. In some implementations, the clasps 230 are barbed clasps that include barbs that increase friction with and/or may partially or completely puncture the leaflets 20, 22. The actuation lines 216 (Figures 43-48) can be actuated separately so that each clasp 230 can be opened and closed separately. Separate operation allows one leaflet 20, 22 to be grasped at a time, or for the repositioning of a clasp 230 on a leaflet 20, 22 that was insufficiently grasped, without altering a successful grasp on the other leaflet 20, 22. The clasps 230 can be fully opened and closed when the inner paddle 222 is not closed, thereby allowing leaflets 20, 22 to be grasped in a variety of positions as the particular situation requires.

Referring now to Figures 22-25, the device 200 is shown in a closed position. When closed, the inner paddles 222 are disposed between the outer paddles 220 and the coaptation element 210. The clasps 230 are disposed between the inner paddles 222 and the coaptation element 210. Upon successful capture of native leaflets 20, 22 the device 200 is moved to and retained in the closed position so that the leaflets 20, 22 are secured within the device 200 by the clasps 230 and are pressed against the coaptation element 210 by the paddles 220, 222. The outer paddles 220 can have a wide curved shape that fits around the curved shape of the coaptation element 210 to more securely grip the leaflets 20, 22 when the device 200 is closed (e.g., as can be seen in Figure 51). The curved shape and rounded edges of the outer paddle 220 also prohibits or inhibits tearing of the leaflet tissue.

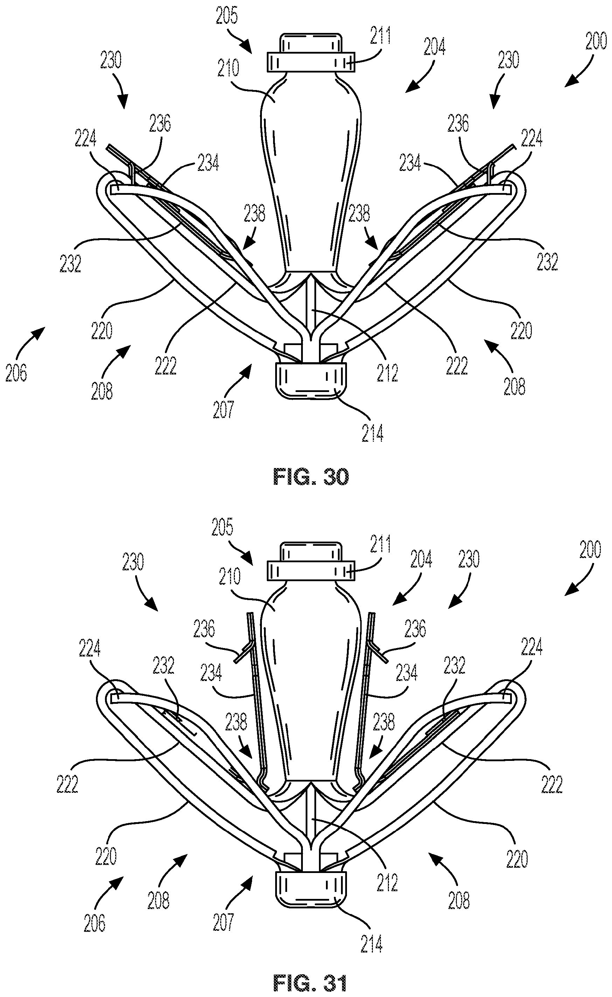

Referring now to Figures 30-37, the implantable device or implant 200 described above is shown in various positions and configurations ranging from partially open to fully open. The paddles 220, 222 of the device 200 transition between each of the positions shown in Figures 30-37 from the closed position shown in Figures 22-25 up extension of the actuation element 212 from a fully retracted to fully extended position.

Referring now to Figures 30-31, the device 200 is shown in a partially open position. The device 200 is moved into the partially open position by extending the actuation element 212. Extending the actuation element 212 pulls down on the bottom portions of the outer paddles 220 and paddle frames 224. The outer paddles 220 and paddle frames 224 pull down on the inner paddles 222, where the inner paddles 222 are connected to the outer paddles 220 and the paddle frames 224. Because the proximal collar 212 (or other attachment element) and coaptation element 210 are held in place by the capture mechanism 213, the inner paddles 222 are caused to articulate, pivot, and/or flex in an opening direction. The inner paddles 222, the outer paddles 220, and the paddle frames all flex to the position shown in Figures 30-31. Opening the paddles 222, 220 and frames 224 forms a gap between the coaptation element 210 and the inner paddle 222 that can receive and grasp the native leaflets 20, 22. This movement also exposes the clasps 230 that can be moved between closed (Figure 30) and open (Figure 31) positions to form a second gap for grasping the native leaflets 20, 22. The extent of the gap between the fixed and moveable arms 232, 234 of the clasp 230 is limited to the extent that the inner paddle 222 has spread away from the coaptation element 210.

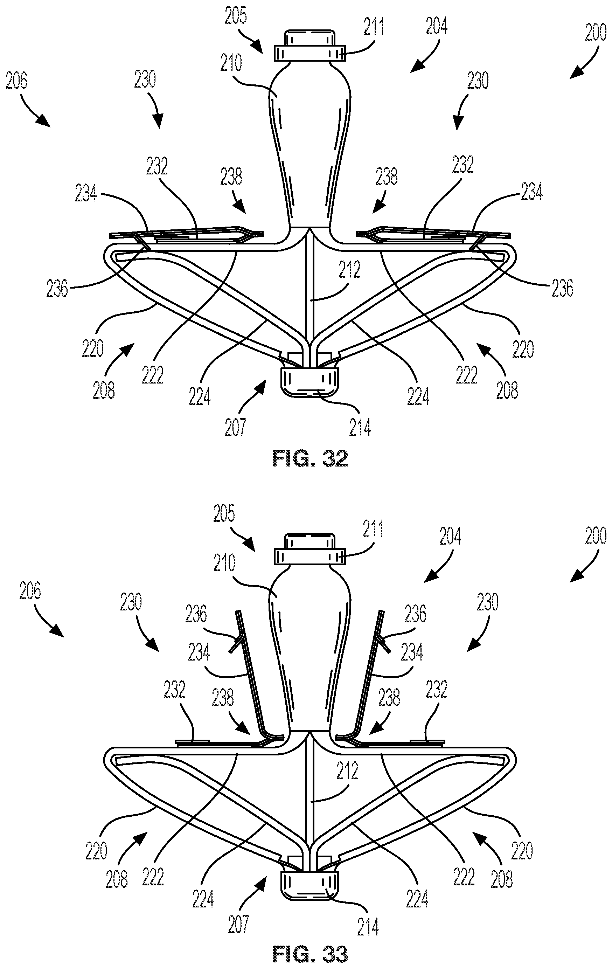

Referring now to Figures 32-33, the device 200 is shown in a laterally extended or open position. The device 200 is moved into the laterally extended or open position by continuing to extend the actuation element 212 described above, thereby increasing the distance between the coaptation element 210 and the cap 214 of the distal portion 207. Continuing to extend the actuation element 212 pulls down on the outer paddles 220 and paddle frames 224, thereby causing the inner paddles 222 to spread apart further from the coaptation element 210. In the laterally extended or open position, the inner paddles 222 extend horizontally more than in other positions of the device 200 and form an approximately 90-degree angle with the coaptation element 210. Similarly, the paddle frames 224 are at their maximum spread position when the device 200 is in the laterally extended or open position. The increased gap between the coaptation element 210 and inner paddle 222 formed in the laterally extended or open position allows clasps 230 to open further (Figure 33) before engaging the coaptation element 210, thereby increasing the size of the gap between the fixed and moveable arms 232, 234.

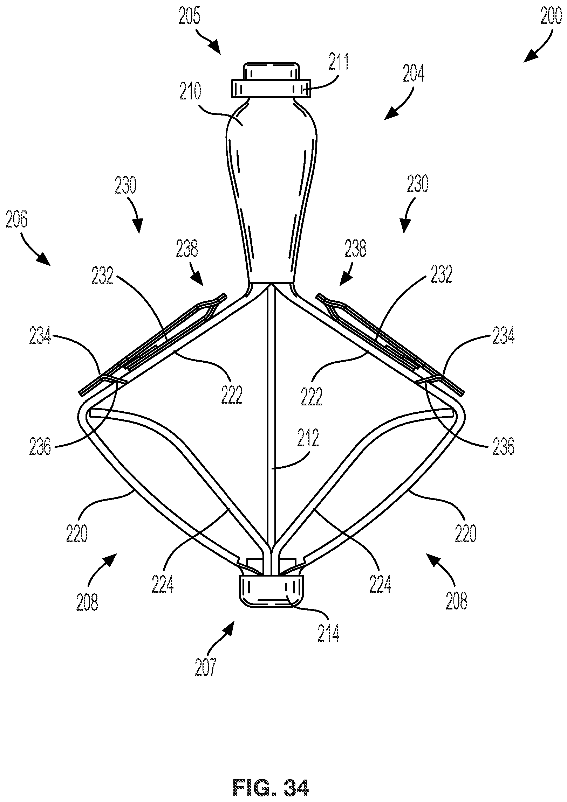

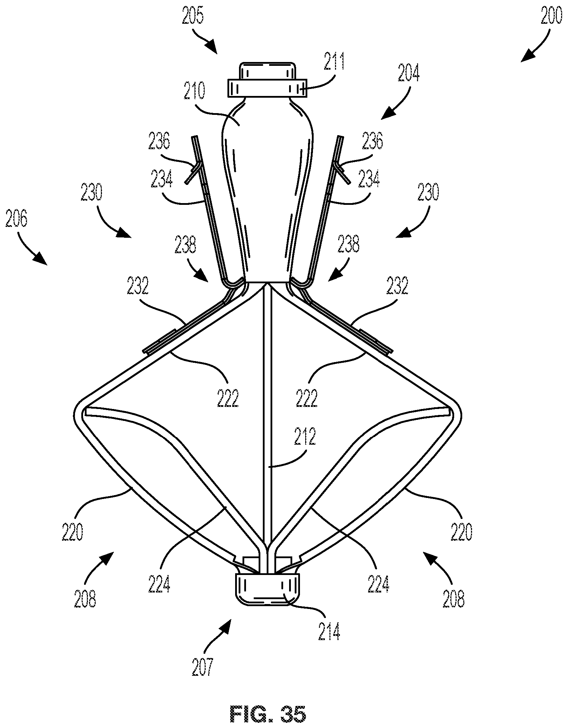

Referring now to Figures 34-35, the example device 200 is shown in a three-quarters extended position. The device 200 is moved into the three-quarters extended position by continuing to extend the actuation element 212 described above, thereby increasing the distance between the coaptation element 210 and the cap 214 of the distal portion 207. Continuing to extend the actuation element 212 pulls down on the outer paddles 220 and paddle frames 224, thereby causing the inner paddles 222 to spread apart further from the coaptation element 210. In the three-quarters extended position, the inner paddles 222 are open beyond 90 degrees to an approximately 135-degree angle with the coaptation element 210. The paddle frames 224 are less spread than in the laterally extended or open position and begin to move inward toward the actuation element 212 as the actuation element 212 extends further. The outer paddles 220 also flex back toward the actuation element 212. As with the laterally extended or open position, the increased gap between the coaptation element 210 and inner paddle 222 formed in the laterally extended or open position allows clasps 230 to open even further (Figure 35), thereby increasing the size of the gap between the fixed and moveable arms 232, 234.

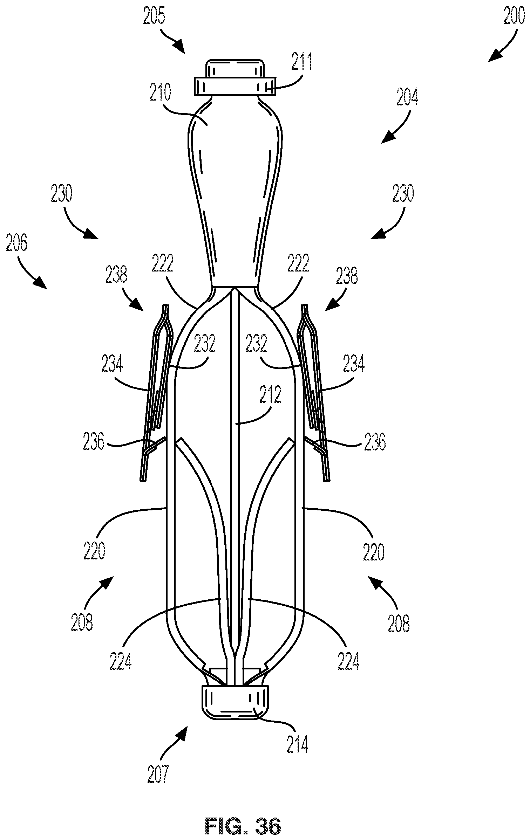

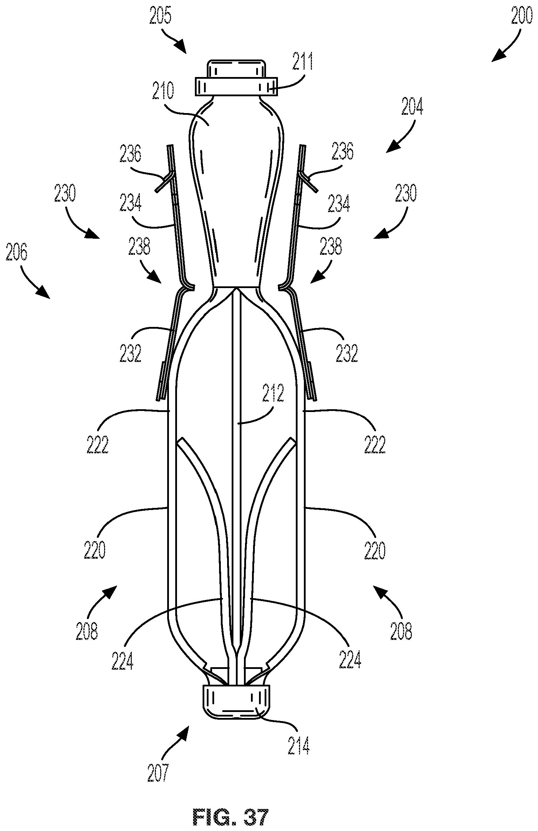

Referring now to Figures 36-37, the example device 200 is shown in a fully extended position. The device 200 is moved into the fully extended position by continuing to extend the actuation element 212 described above, thereby increasing the distance between the coaptation element 210 and the cap 214 of the distal portion 207 to a maximum distance allowable by the device 200. Continuing to extend the actuation element 212 pulls down on the outer paddles 220 and paddle frames 224, thereby causing the inner paddles 222 to spread apart further from the coaptation element 210. The outer paddles 220 and paddle frames 224 move to a position where they are close to the actuation element. In the fully extended position, the inner paddles 222 are open to an approximately 180-degree angle with the coaptation element 210. The inner and outer paddles 222, 220 are stretched straight in the fully extended position to form an approximately 180-degree angle between the paddles 222, 220. The fully extended position of the device 200 provides the maximum size of the gap between the coaptation element 210 and inner paddle 222, and, in some implementations, allows clasps 230 to also open fully to approximately 180 degrees (Figure 37) between the fixed and moveable arms 232, 234 of the clasp 230. The position of the device 200 is the longest and the narrowest configuration. Thus, the fully extended position of the device 200 may be a desirable position for bailout of the device 200 from an attempted implantation or may be a desired position for placement of the device in a delivery catheter, or the like.

Configuring the prosthetic device or implant 200 such that the anchors 208 can extend to a straight or approximately straight configuration (e.g. approximately 120-180 degrees relative to the coaptation element 210) can provide several advantages. For example, this configuration can reduce the radial crimp profile of the prosthetic device or implant 200. It can also make it easier to grasp the native leaflets 20, 22 by providing a larger opening between the coaptation element 210 and the inner paddles 222 in which to grasp the native leaflets 20, 22. Additionally, the relatively narrow, straight configuration can prevent or reduce the likelihood that the prosthetic device or implant 200 will become entangled in native anatomy (e.g., chordae tendineae CT shown in Figures 3 and 4) when positioning and/or retrieving the prosthetic device or implant 200 into the delivery system 202.

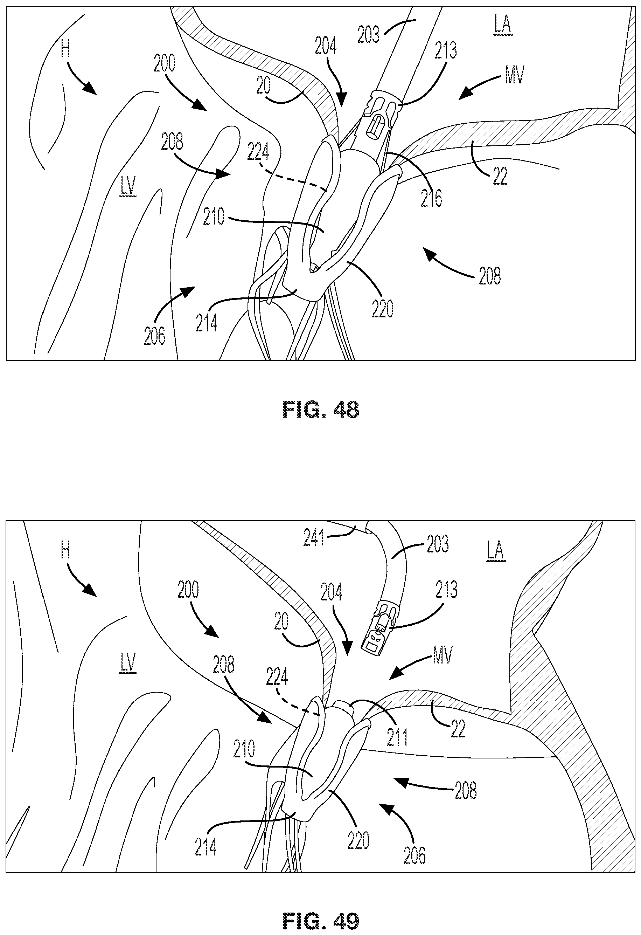

Referring now to Figures 38-49, an example implantable device 200 is shown being delivered and implanted within the native valve (e.g., mitral valve MV) of the heart H. As described above, the device 200 shown in Figures 38-49 includes the optional covering 240 (e.g., Figure 25) over the coaptation element 210, clasps 230, inner paddles 222 and/or the outer paddles 220. The device 200 is deployed from a delivery system (e.g., which can comprise an implant catheter 203 that is extendable from a steerable catheter 241 and/or a delivery catheter/sheath 242). A capture mechanism 213 is attached to the distal end of the implant catheter 202. The device 200 can be retained by the capture mechanism 213 (see e.g., Figures 43 and 48) and actuated by extending or retracting the actuation element 212. Fingers of the capture mechanism 213 removably attach the collar 211 to the delivery system. In some implementations, the capture mechanism 213 is held closed around the collar 211 by the actuation element 212, such that removal of the actuation element 212 allows the fingers of the capture mechanism 213 to open and release the collar 211 to decouple the capture mechanism 213 from the device 200 after the device 200 has been successfully implanted.

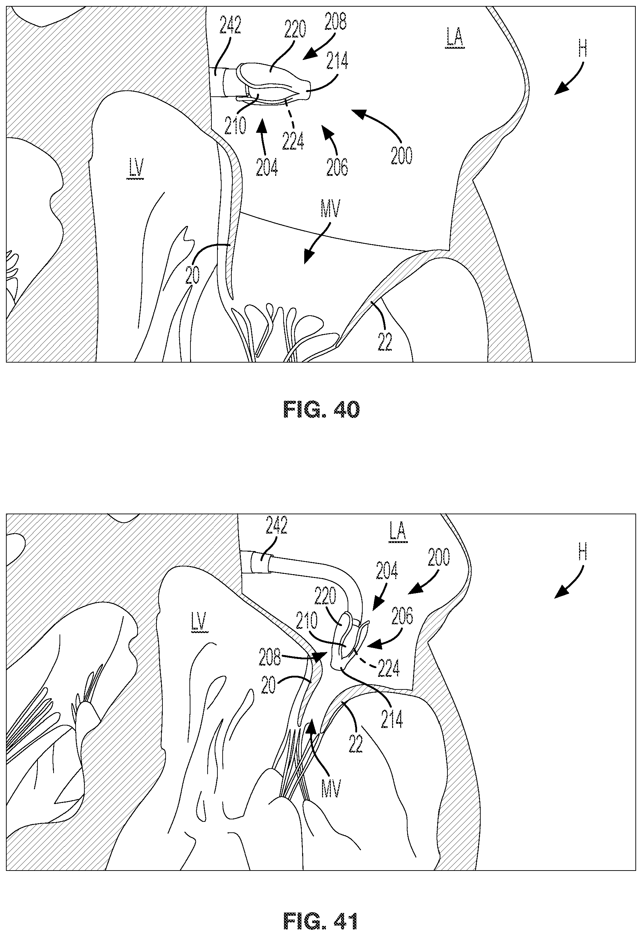

Referring now to Figure 38, the delivery system (e.g., a delivery catheter/sheath 242 thereof) is inserted into the left atrium LA through the septum and the device/implant 200 is deployed from the delivery system (e.g., an implant catheter 203 retaining the device/implant 200 can be extended to deploy the device/implant 200 out from a steerable catheter 241) in the fully open condition for the reasons discussed above with respect to the device 100. The actuation element 212 is then retracted to move the device 200 through the partially closed condition (Figure 39) and to the fully closed condition shown in Figures 40-41. Then the delivery system or catheter maneuvers the device/implant 200 towards the native valve as shown in Figure 41. Referring now to Figure 42, when the device 200 is aligned with the native valve, the actuation element 212 is extended to open the paddles 220, 222 into the partially opened position and the actuation lines 216 (Figures 43-48) are retracted to open the clasps 230 to prepare for leaflet grasp. Next, as shown in Figures 43-44, the partially open device 200 is inserted through the native valve (e.g., by advancing an implant catheter 203 from a steerable catheter 241) until leaflets 20, 22 are properly positioned in between the inner paddles 222 and the coaptation element 210 and inside the open clasps 230.

Figure 45 shows the device 200 with both clasps 230 closed, though the barbs 236 of one clasp 230 missed one leaflet 22. As can be seen in Figures 45-47, the out of position clasp 230 is opened and closed again to properly grasp the missed leaflet 22. When both leaflets 20, 22 are grasped properly, the actuation element 212 is retracted to move the device 200 into the fully closed position shown in Figure 48. With the device 200 fully closed and implanted in the native valve, the actuation element 212 is disengaged from the cap 214 and is withdrawn to release the capture mechanism 213 from the proximal collar 211 (or other attachment element) so that the capture mechanism 213 can be withdrawn into the delivery system (e.g., into a steerable catheter 241 and/or a delivery catheter/sheath 242), as shown in Figure 49. Once deployed, the device 200 can be maintained in the fully closed position with a mechanical means such as a latch or may be biased to remain closed through the use of spring material, such as steel, and/or shape-memory alloys such as Nitinol. For example, the paddles 220, 222 can be formed of steel or Nitinol shape-memory alloy-produced in a wire, sheet, tubing, or laser sintered powder-and are biased to hold the outer paddles 220 closed around the inner paddles 222, coaptation element 210, and/or the clasps 230 pinched around native leaflets 20, 22.

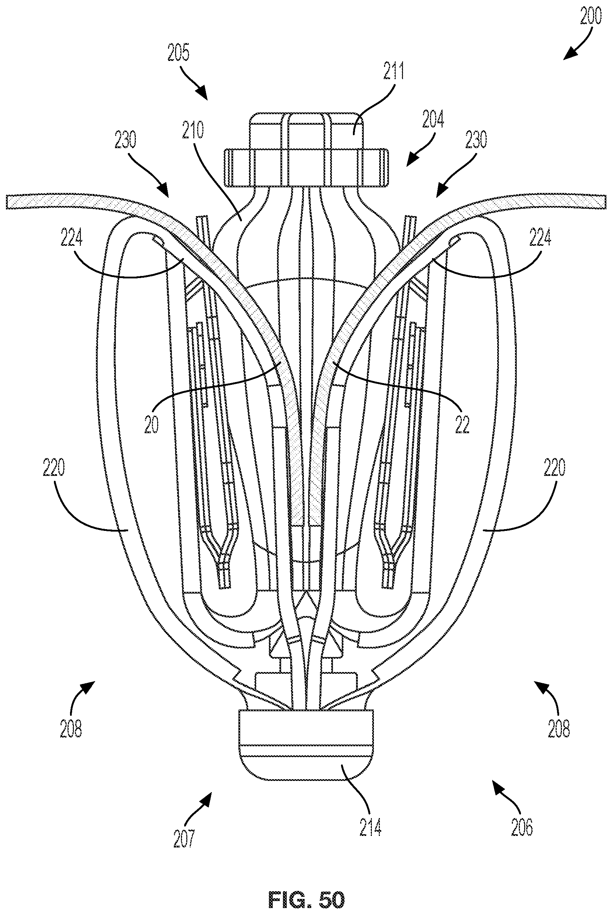

Referring to Figures 50-54, once the device 200 is implanted in a native valve, the coaptation element 210 functions as a gap filler in the valve regurgitant orifice, such as the gap 26 in the mitral valve MV illustrated by Figure 6 or a gap in another native valve. In some implementations, when the device 200 has been deployed between the two opposing valve leaflets 20, 22, the leaflets 20, 22 no longer coapt against each other in the area of the coaptation element 210, but instead coapt against the coaptation element 210. This reduces the distance the leaflets 20, 22 need to be approximated to close the mitral valve MV during systole, thereby facilitating repair of functional valve disease that may be causing mitral regurgitation. A reduction in leaflet approximation distance can result in several other advantages as well. For example, the reduced approximation distance required of the leaflets 20, 22 reduces or minimizes the stress experienced by the native valve. Shorter approximation distance of the valve leaflets 20,22 can also require less approximation forces which can result in less tension experienced by the leaflets 20, 22 and less diameter reduction of the valve annulus. The smaller reduction of the valve annulus-or none at all-can result in less reduction in valve orifice area as compared to a device without a coaptation element or spacer. In this way, the coaptation element 210 can reduce the transvalvular gradients.

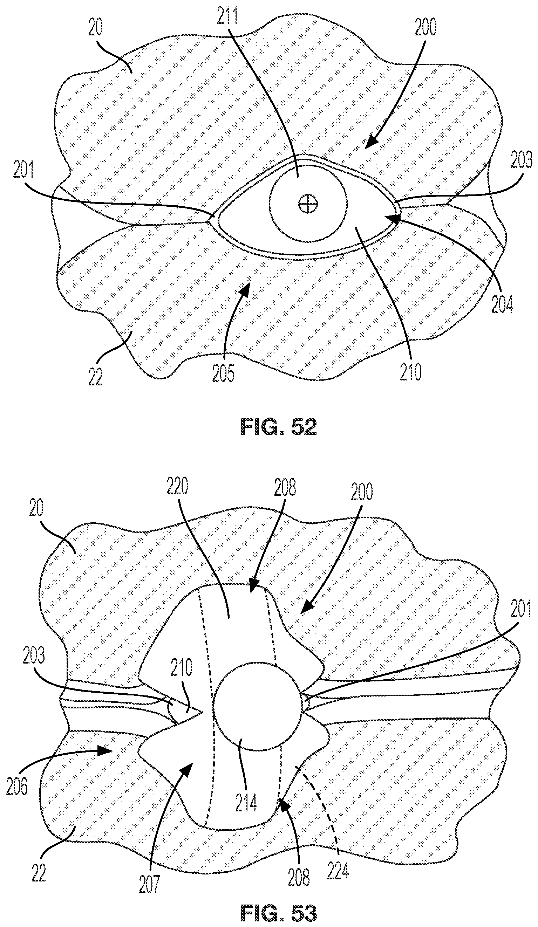

To adequately fill the gap 26 between the leaflets 20, 22, the device 200 and the components thereof can have a wide variety of different shapes and sizes. For example, the outer paddles 220 and paddle frames 224 can be configured to conform to the shape or geometry of the coaptation element 210 as is shown in Figures 50-54. As a result, the outer paddles 220 and paddle frames 224 can mate with both the coaptation element 210 and the native valve leaflets 20, 22. In some implementations, when the leaflets 20, 22 are coapted against the coaptation element 210, the leaflets 20, 22 fully surround or "hug" the coaptation element 210 in its entirety, thus small leaks at lateral and medial aspects 201, 203 of the coaptation element 210 can be prevented. The interaction of the leaflets 20, 22 and the device 200 is made clear in Figure 51, which shows a schematic atrial or surgeon's view that shows the paddle frame 224 (which would not actually be visible from a true atrial view, e.g., Figure 52), conforming to the coaptation element 210 geometry. The opposing leaflets 20, 22 (the ends of which would also not be visible in the true atrial view, e.g., Figure 52) being approximated by the paddle frames 224, to fully surround or "hug" the coaptation element 210.

This coaptation of the leaflets 20, 22 against the lateral and medial aspects 201, 203 of the coaptation element 210 (shown from the atrial side in Figure 52, and the ventricular side in Figure 53) would seem to contradict the statement above that the presence of a coaptation element 210 minimizes the distance the leaflets need to be approximated. However, the distance the leaflets 20, 22 need to be approximated is still minimized if the coaptation element 210 is placed precisely at a regurgitant gap 26 and the regurgitant gap 26 is less than the width (medial-lateral) of the coaptation element 210.

Figure 50 illustrates the geometry of the coaptation element 210 and the paddle frame 224 from an LVOT perspective. As can be seen in this view, the coaptation element 210 has a tapered shape being smaller in dimension in the area closer to where the inside surfaces of the leaflets 20, 22 are required to coapt and increase in dimension as the coaptation element 210 extends toward the atrium. Thus, the depicted native valve geometry is accommodated by a tapered coaptation element geometry. Still referring to Figure 50, the tapered coaptation element geometry, in conjunction with the illustrated expanding paddle frame 224 shape (toward the valve annulus) can help to achieve coaptation on the lower end of the leaflets, reduce stress, and minimize transvalvular gradients.

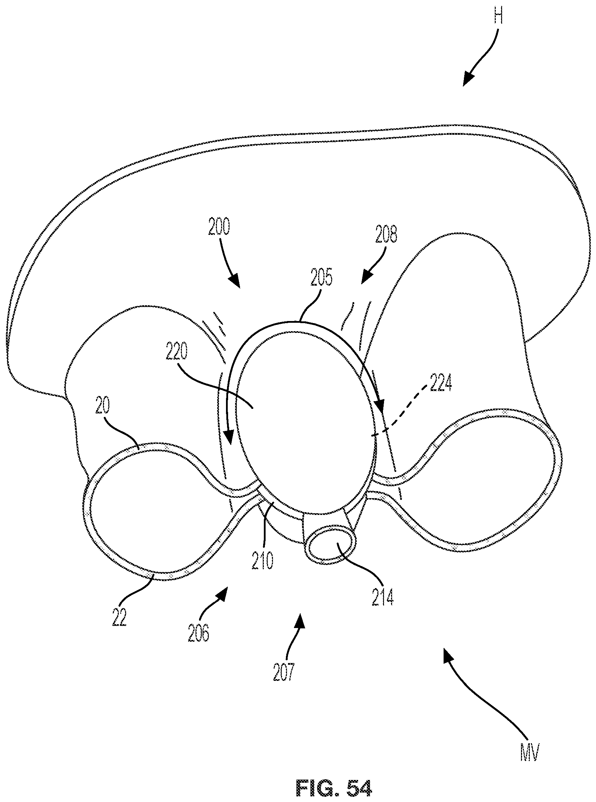

Referring to Figure 54, the shape of the coaptation element 210 and the paddle frames 224 can be defined based on an Intra-Commissural view of the native valve and the device 210. Two factors of these shapes are leaflet coaptation against the coaptation element 210 and reduction of stress on the leaflets due to the coaptation. Referring to Figures 54 and 24, to both coapt the valve leaflets 20, 22 against the coaptation element 210 and reduce the stress applied to the valve leaflets 20, 22 by the coaptation element 210 and/or the paddle frames 224, the coaptation element 210 can have a round or rounded shape and the paddle frames 224 can have a full radius that spans nearly the entirety of the paddle frame 224. The round shape of the coaptation element 210 and/or the illustrated fully rounded shape of the paddle frames 224 distributes the stresses on the leaflets 20, 22 across a large, curved engagement area 205. For example, in Figure 54, the force on the leaflets 20, 22 by the paddle frames is spread along the entire rounded length of the paddle frame 224, as the leaflets 20 try to open during the diastole cycle.

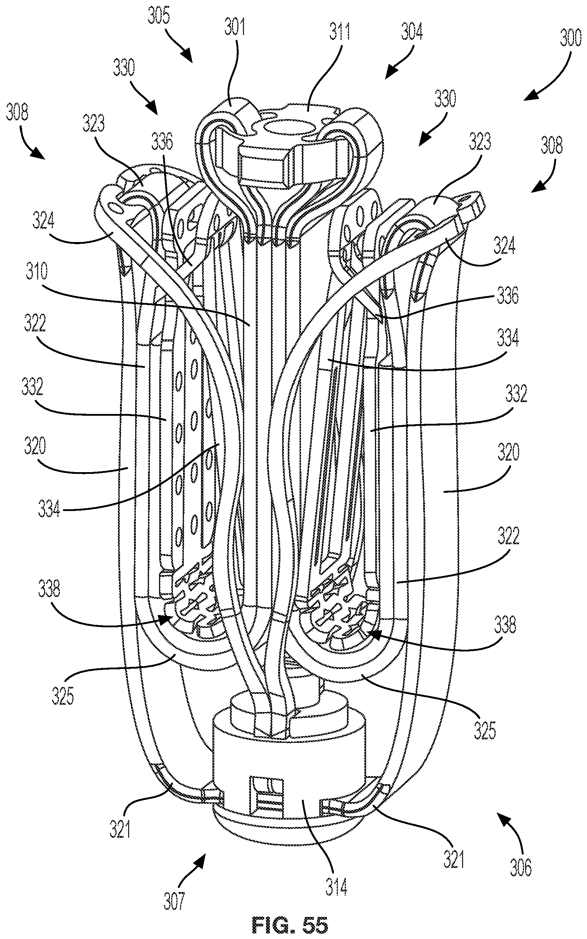

Referring now to Figure 55, an example of an implantable prosthetic device or implant 300 is shown. The implantable device 300 is one of the many different configurations that the device 100 that is schematically illustrated in Figures 8-14 can take. The device 300 can include any other features for an implantable device or implant discussed in the present application, and the device 300 can be positioned to engage valve tissue 20, 22 as part of any suitable valve repair system (e.g., any valve repair system disclosed in the present application).

The implantable device or implant 300 includes a proximal or attachment portion 305, an anchor portion 306, and a distal portion 307. In some implementations, the device/implant 300 includes a coaptation portion 304, and the coaptation portion 304 can optionally include a coaptation element 310 (e.g., spacer, plug, membrane, sheet, etc.) for implantation between the leaflets 20, 22 of the native valve. In some implementations, the anchor portion 306 includes a plurality of anchors 308. In some implementations, each anchor 308 can include one or more paddles, e.g., outer paddles 320, inner paddles 322, paddle extension members or paddle frames 324. The anchors can also include and/or be coupled to clasps 330. In some implementations, the attachment portion 305 includes a first or proximal collar 311 (or other attachment element) for engaging with a capture mechanism (e.g., a capture mechanism such as the capture mechanism 213 shown in Figures 43-49) of a delivery system (e.g., a delivery system such as the system shown in Figures 38-42 and 49).

The anchors 308 can be attached to the other portions of the device and/or to each other in a variety of different ways (e.g., directly, indirectly, welding, sutures, adhesive, links, latches, integrally formed, a combination of some or all of these, etc.). In some implementations, the anchors 308 are attached to a coaptation member or coaptation element 310 by connection portions 325 and to a cap 314 by connection portions 321.

The anchors 308 can comprise first portions or outer paddles 320 and second portions or inner paddles 322 separated by connection portions 323. The connection portions 323 can be attached to paddle frames 324 that are hingeably attached to a cap 314 or other attachment portion. In this manner, the anchors 308 are configured similar to legs in that the inner paddles 322 are like upper portions of the legs, the outer paddles 320 are like lower portions of the legs, and the connection portions 323 are like knee portions of the legs.

In implementations with a coaptation member or coaptation element 310, the coaptation member or coaptation element 310 and the anchors 308 can be coupled together in various ways. For example, as shown in the illustrated implementation, the coaptation element 310 and the anchors 308 can be coupled together by integrally forming the coaptation element 310 and the anchors 308 as a single, unitary component. This can be accomplished, for example, by forming the coaptation element 310 and the anchors 308 from a continuous strip 301 of a braided or woven material, such as braided or woven nitinol wire. In the illustrated example, the coaptation element 310, the outer paddle portions 320, the inner paddle portions 322, and the connection portions 321, 323, 325 are formed from the continuous strip of fabric 301.

Like the anchors 208 of the implantable device or implant 200 described above, the anchors 308 can be configured to move between various configurations by axially moving the distal end of the device (e.g., cap 314, etc.) relative to the proximal end of the device (e.g., proximal collar 311 or other attachment element, etc.) and thus the anchors 308 move relative to a midpoint of the device. This movement can be along a longitudinal axis extending between the distal end (e.g., cap 314, etc.) and the proximal end (e.g., collar 311 or other attachment element, etc.) of the device. For example, the anchors 308 can be positioned in a fully extended or straight configuration (e.g., similar to the configuration of device 200 shown in Figure 36) by moving the distal end (e.g., cap 314, etc.) away from the proximal end of the device.

In some implementations, in the straight configuration, the paddle portions 320, 322 are aligned or straight in the direction of the longitudinal axis of the device. In some implementations, the connection portions 323 of the anchors 308 are adjacent the longitudinal axis of the coaptation element 310 (e.g., similar to the configuration of device 200 shown in Figure 36). From the straight configuration, the anchors 308 can be moved to a fully folded configuration (e.g., Figure 55), e.g., by moving the proximal end and distal end toward each other and/or toward a midpoint or center of the device. Initially, as the distal end (e.g., cap 314, etc.) moves toward the proximal end and/or midpoint or center of the device, the anchors 308 bend at connection portions 321, 323, 325, and the connection portions 323 move radially outwardly relative to the longitudinal axis of the device 300 and axially toward the midpoint and/or toward the proximal end of the device (e.g., similar to the configuration of device 200 shown in Figure 34). As the cap 314 continues to move toward the midpoint and/or toward the proximal end of the device, the connection portions 323 move radially inwardly relative to the longitudinal axis of the device 300 and axially toward the proximal end of the device (e.g., similar to the configuration of device 200 shown in Figure 30).

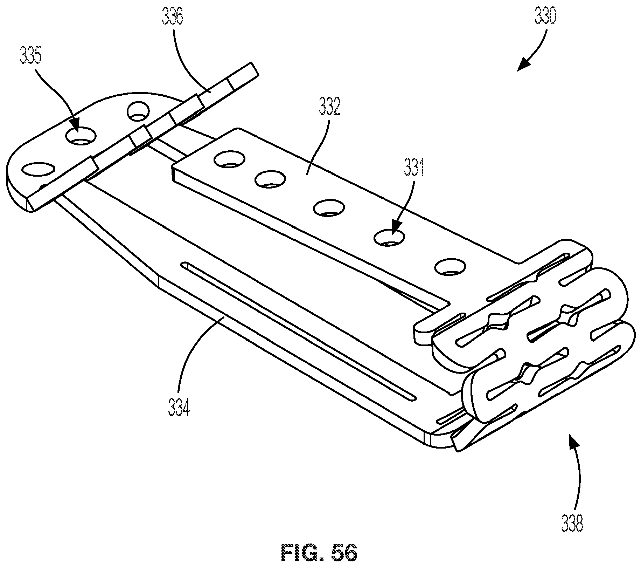

In some implementations, the clasps comprise a moveable arm coupled to an anchor. In some implementations, the clasps 330 (as shown in detail in Figure 56) include a base or fixed arm 332, a moveable arm 334, optional barbs/friction-enhancing elements 336, and a joint portion 338. The fixed arms 332 are attached to the inner paddles 322, with the joint portion 338 disposed proximate the coaptation element 310. The joint portion 338 is spring-loaded so that the fixed and moveable arms 332, 334 are biased toward each other when the clasp 330 is in a closed condition.

The fixed arms 332 are attached to the inner paddles 322 through holes or slots 331 with sutures (not shown). The fixed arms 332 can be attached to the inner paddles 322 with any suitable means, such as screws or other fasteners, crimped sleeves, mechanical latches or snaps, welding, adhesive, or the like. The fixed arms 332 remain substantially stationary relative to the inner paddles 322 when the moveable arms 334 are opened to open the clasps 330 and expose the barbs 336. The clasps 330 are opened by applying tension to actuation lines (e.g., the actuation lines 216 shown in Figures 43-48) attached to holes 335 in the moveable arms 334, thereby causing the moveable arms 334 to articulate, pivot, and/or flex on the joint portions 338.

In short, the implantable device or implant 300 is similar in configuration and operation to the implantable device or implant 200 described above, except that the coaptation element 310, outer paddles 320, inner paddles 322, and connection portions 321, 323, 325 are formed from the single strip of material 301. In some implementations, the strip of material 301 is attached to the proximal collar 311, cap 314, and paddle frames 324 by being woven or inserted through openings in the proximal collar 311, cap 314, and paddle frames 324 that are configured to receive the continuous strip of material 301. The continuous strip 301 can be a single layer of material or can include two or more layers. In some implementations, portions of the device 300 have a single layer of the strip of material 301 and other portions are formed from multiple overlapping or overlying layers of the strip of material 301.

For example, Figure 55 shows a coaptation element 310 and inner paddles 322 formed from multiple overlapping layers of the strip of material 301. The single continuous strip of material 301 can start and end in various locations of the device 300. The ends of the strip of material 301 can be in the same location or different locations of the device 300. For example, in the illustrated example of Figure 55, the strip of material 301 begins and ends in the location of the inner paddles 322.

As with the implantable device or implant 200 described above, the size of the coaptation element 310 can be selected to minimize the number of implants that a single patient will require (preferably one), while at the same time maintaining low transvalvular gradients. In particular, forming many components of the device 300 from the strip of material 301 allows the device 300 to be made smaller than the device 200. For example, In some implementations, the anterior-posterior distance at the top of the coaptation element 310 is less than 2 mm, and the medial-lateral distance of the device 300 (i.e., the width of the paddle frames 324 which are wider than the coaptation element 310) at its widest is about 5 mm.

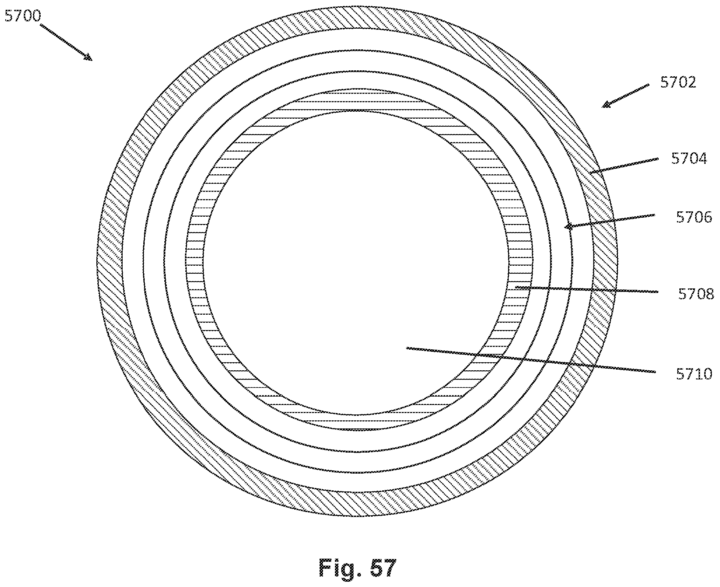

Figures 57-65B illustrate example implementations of implant catheters that can be used as the implant catheters 103, 203 described above. As is illustrated in Figures 16-20 and 38-49, implant catheters 103, 203 can enter the right atrium from the IVC or SVC and can, optionally, enter the left atrium from the right atrium. The implant catheters 103, 203 can be flexed downward in order to properly place an implantable prosthetic device 100, 200. In an example implementation, the implant catheter 103 is enclosed in a steerable catheter 140, which is itself enclosed in a guide sheath 142 as described above. Similarly, the implant catheter 203 is enclosed in a steerable catheter 241, which is itself enclosed in a guide sheath 242 as described above. In order to be flexible enough to form the bends illustrated in Figures 18 and 41, and 42, while still resisting compression along its length, a catheter with a non-compressible but flexible portion is required.

Figure 57 illustrates a cross-section of such a flexible, non-compressible catheter 5700. As shown, an example implementation of such a catheter is comprised of a number of layers 5702. Some layers 5702 flow into other layers when the flexible, non-compressible catheter 5700 is made by melting or partially melting some of the layers to bond the layers together. This is known as "reflowing". However, in the illustrations of Figures 57-59 the layers are shown discretely to simplify the drawings.

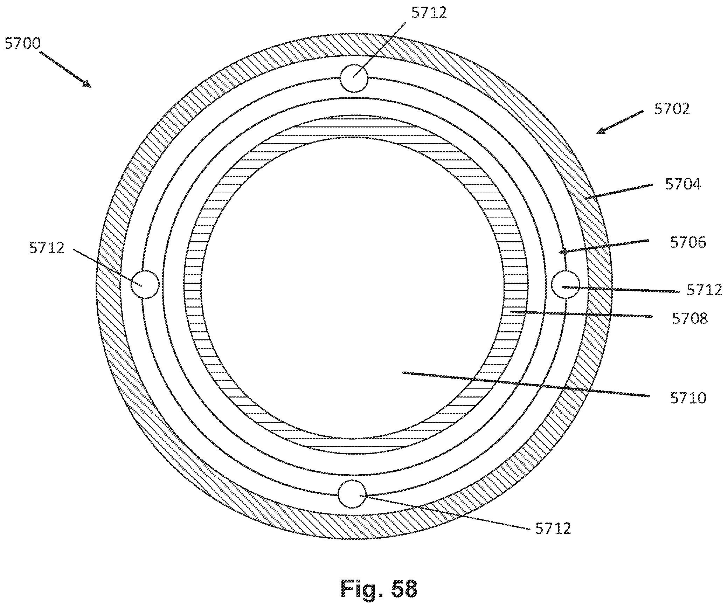

Still Referring to Figure 57, the flexible, non-compressible catheter 5700 can include an outer layer (referred to as a "jacket") 5704, various intermediate layers 5706, and an inner liner 5708. As will be described in greater detail herein, the intermediate layers 5706 can be selected to provide characteristics such as rigidity/flexibility, and resistance to compression. In addition to the main lumen 5710, additional sub-lumens can be added to implementations of the flexible, non-compressible catheter 5700. An example of this is shown in Figure 58 where four sub-lumens 5712 are positioned amongst the intermediate layers 5706. The number and location of the sub-lumens 5712 are non-limiting examples. A greater or fewer number of sub-lumens can be formed in example implementations. Additionally, the location of the sub-lumens 5712 can vary depending upon the intended use of the catheter 5700. The sub-lumens can be used for clasp actuation lines, tethers, pressure monitoring, and other device control and/or monitoring,

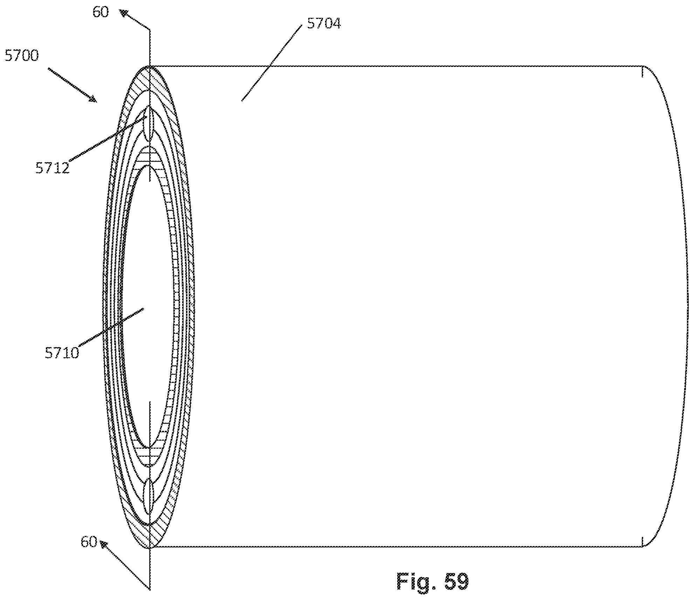

Figure 59 illustrates a side perspective view of an example implementation. Ordinarily, the catheter 5700 is formed with a round cross-section but other implementations can also be formed in other shapes such as, without limitation, oval and teardrop shaped. Shown is the main lumen 5710 with a single sub-lumen 5712, but any number of sub-lumens can be included.

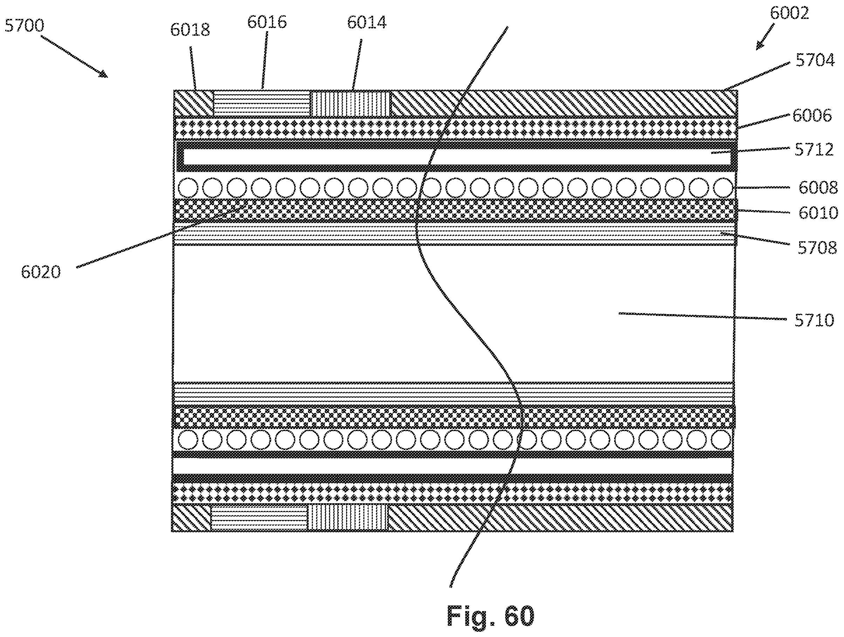

Figure 60 is a cross-sectional view taken along the plane indicated by lines 60-60 in Figure 59. The various layers 5702 forming the catheter 5700 are shown in the catheter cross-section illustrated in Figure 60. In the illustrated example, the upper and lower portions of the figure are symmetrical and thus, only the upper portion is annotated with reference numbers. However, in some implementations, the upper and lower portions of the cross-section are not symmetrical.

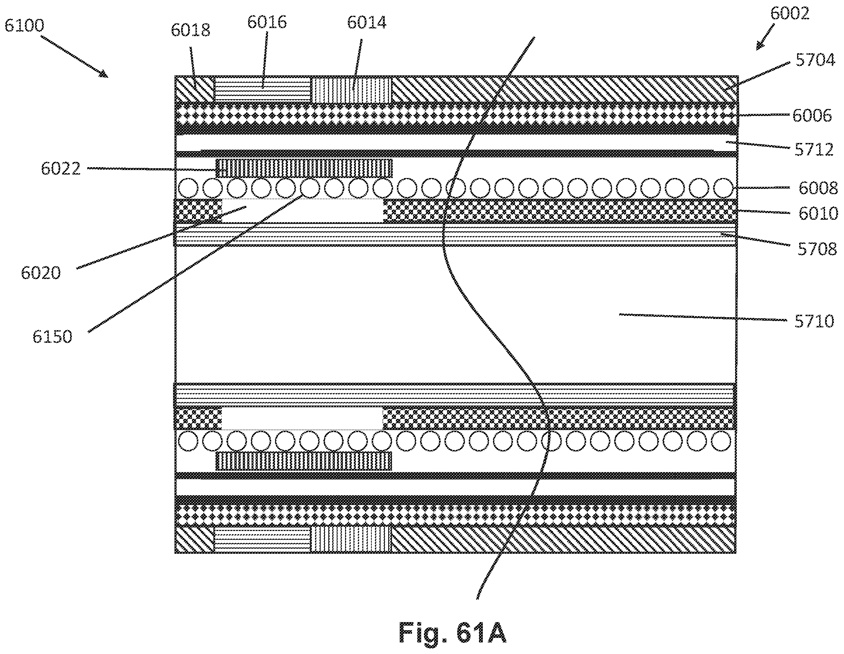

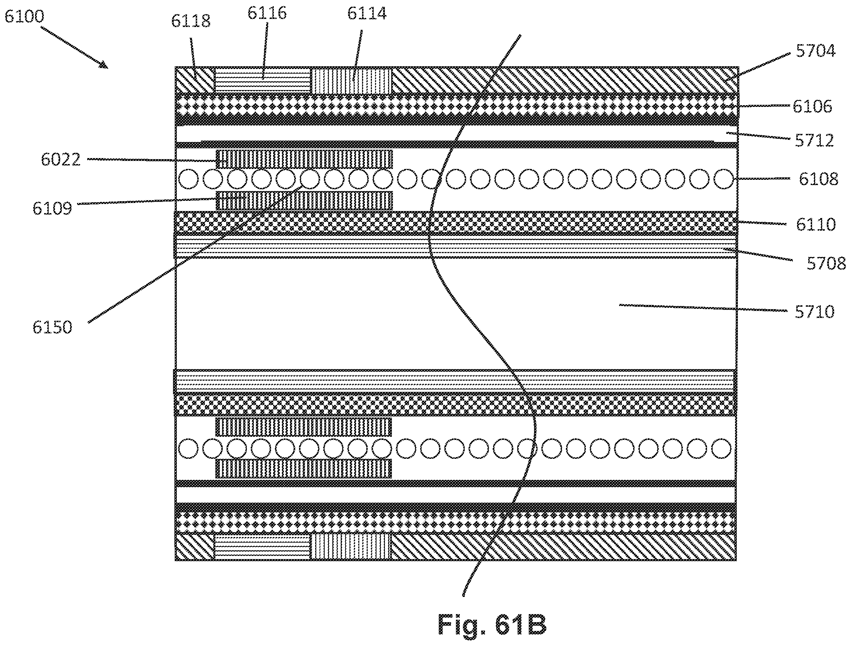

The right-hand side of Figure 60 illustrates the proximal end of the implant catheter 5700. The curved line through the catheters in Figures 60, 61A and 61B represents the catheter being significantly longer than shown in the Figures. The right hand or proximal side of jacket 5704 can be formed from a NYLON material. Some example implementations of the proximal jacket portion 5704 can be formed from materials with a lower durometer than NYLON. Examples of materials that have a lower durometer than nylon include, but are not limited to, silicone rubber latex, thermoplastic elastomers including, but not limited to, PEBAX, silicone, polyvinylchloride (PVC), polyethylene (PE), Polytetrafluoroethylene (PTFE - Teflon), silicone, polyurethane (PU) and blends of one or more of NYLON, thermoplastic elastomers including, but not limited to, PEBAX, silicone, PVC, Dacron, PE, PTFE, polyurethane (PU) and silicone.

Referring to the left or distal side of Figure 60, the distal end of the jacket can optionally have a varying durometer. In some implementations, the entire jacket 5704 can be made from the same material. Providing the distal end of the jacket with a varying durometer enhances the ability of the catheter 5700 to be bent to the positions illustrated by Figures 18-20. The jacket 5704 can be provided with a distal section having a varying durometer in a wide variety of different ways. In the illustrated example, the outer jacket 504 has distal sections 6014, 6016, 6018 with different durometers. The sections 6014, 6016, 6018 can be formed with different durometers in a variety of different ways. For example, the sections can be made from different materials, the sections can be reinforced to different degrees, the sections can include cuts or reliefs, etc. In one example implementation, the sections 6014, 6016, 6018 each comprise a thermoplastic elastomer, such as PEBAX. For example, the first section 6014 can have a durometer between 45D and 70D, such as about 55D and can be made from a thermoplastic elastomer, such as Pebax, the second section 6016 can have a durometer between 15D and 35D, such as about 25D and can be made from a thermoplastic elastomer, such as Pebax, and the third section 6018 can have a durometer between 45D and 70D, such as about 55D and can be made from a thermoplastic elastomer, such as Pebax. These modifications serve to form a section of jacket that is more flexible than the remainder of the jacket 6104. The thermoplastic elastomer materials, such as Pebax, and the durometer ranges are only examples, and other materials, as well as greater or fewer sections, can be used in other implementations.

In the example implementation illustrated by Figure 60, the next inner layer is a braid or mesh layer 6006 that can be used to provide resistance to twisting to permit torque to be applied by the catheter in order to manipulate, install, or otherwise position certain tools and/or implantable devices using the catheter 5700. The braid layer 6006 can be woven from a plurality of individual metal strands. In some example implementations, instead of being woven, the braid layer 6006 can be formed from one or more laser cut tubes. The cuts in the tube are also configured to provide resistance to twisting to permit torque to be applied by the catheter in order to manipulate, install, or otherwise position certain tools or implantable devices using the catheter 5700, with the advantage that the cut pattern can be varied along the length of the catheter and can be optimized for the specific application and location of the catheter.

In the example implementation illustrated by Figure 60, one or more lumens 5712 are disposed inside the braid or mesh layer 6006. In some example implementations, the lumens 5712 are omitted. The one or more lumens can take a wide variety of different forms. In one example implementation, the one or more lumens are formed by positioning a tube inside the braid or mesh layer 6006. Such a tube can be made from a wide variety of different materials. In one example implementation, the tube that forms the lumen 5712 is made from a material that has a melt or flow temperature that is higher than the melt or flow temperature of the material of the outer jacket 5704. In one example implementation, the one or more lumens 5712 are formed from PTFE. For example, the one or more lumens can be formed from a PTFE tube or jacket having a durometer of between 45D and 65D, such as about 55D.

Still referring to Figure 60, the next inner layer is a coil 6008. The coil can take a wide variety of different forms. The coil 6008 can be made from round wire, square wire, rectangular wire, or other shaped wire, as will be described in more detail below. In one example implementation, the coil 6008 is formed from round wire having a diameter from 0.0508 mm to 0.508 mm (0.002 to 0.020 inches), such as from 1.016 mm to about 0.254 mm (0.04 to about 0.010 inches), such as from 1.524 mm to 1.778 mm (0.060 to 0.070 inches), such as about 0.1651 mm (0.0065 inches). The coil provides resistance to compression of the catheter 5700, while still allowing the catheter to flex. The coil 6008 permits the catheter to be inserted into a steerable catheter 140 (See Figure 16) accurately (without risk of expansion or contraction along the length of the lumen) extended or retracted from the steerable catheter.

Still referring to Figure 60, the next inner layer is a wall 6010. The wall 6010 can take a wide variety of different forms. The wall 6010 can be formed from a NYLON material. In some implementations, the wall 6010 can be formed from materials with a lower durometer than NYLON. Examples of materials that have a lower durometer than nylon include, but are not limited to, silicone rubber latex, thermoplastic elastomers including, but not limited to, PEBAX silicone, polyvinylchloride (PVC), polyethylene (PE), Polytetrafluoroethylene (PTFE - Teflon), silicone, polyurethane (PU) and blends of one or more of NYLON, thermoplastic elastomers including, but not limited to PEBAX, silicone, PVC, Dacron, PE, PTFE, polyurethane (PU) and silicone. In an example implementation, the wall 6010 has a thickness range from 0.00635 mm to 0.2032 mm (0.00025 to 0.008 inches), such as 0.0127 mm to 0.1016 mm (0.0005 to 0.004 inches), such as 0.0254 mm to 0.0508 mm (0.001 to 0.002 inches).

Referring to the left or distal side of Figure 60, the distal end of the wall 6010 can optionally have a reduced durometer compared to the remainder of the wall. Providing the distal end of the wall 6010 with a lower durometer enhances the ability of the catheter 5700 to be bent to the positions illustrated by Figures 18-20. The wall 6010 can be provided with a distal section having a reduced durometer in a wide variety of different ways. For example, the distal end of the wall 6010 can be made from a different material than the remainder of the wall, can include cuts or reliefs, etc. In one example implementation, the distal end of the catheter can comprise a thermoplastic elastomer, such as PEBAX. This modification serves to form a section of the wall 6010 that is more flexible than the remainder of the wall 6010. The thermoplastic elastomer materials, such as Pebax, and the durometer ranges are only non-limiting examples, and other materials can be used in other implementations.

Still referring to Figure 60, the innermost layer of the catheter 5700 is the wall 5708 of the main lumen 5710. The main lumen 5710 can take a wide variety of different forms. In one example implementation, the main lumen 5710 is formed placing or forming the other layers 6010, 6008, 6006, 5704 around a tube or annular wall 5708. Such a tube can be made from a wide variety of different materials. In one example implementation, the tube 5708 is made from a material that has a melt or flow temperature that is higher than the melt or flow temperature of the material of the wall 6010. In one example implementation, the tube 5708 is formed from PTFE.

In one example implementation, the layers of the catheter 5700 are assembled as illustrated by Figure 60 and heated to cause one or more of the layers 5702 to melt and flow. For example, the layers 5704 and 6010 and the portions 6014, 6016, 6018 melt, flow through the coil 6008 and the braid 6006, blend together where they meet, and solidify to form the completed, composite catheter 5700. As mentioned above, this process is sometimes referred to as "reflowing."

The catheter structure illustrated by Figure 60 provides the capability to transmit torque without twisting or distortion as a result of the braid 6006 and resistance to expansion and contraction along its length as a result of the coil 6008. However, one effect of the reflowing process is that the reflowed plastic material fills spaces between the individual windings of the coil 6008 and can bond the individual windings together, making the catheter less flexible. As mentioned above, the distal end of the catheter 5700 is caused to undergo a relatively sharp turn or bend when delivering an implant 100 (See Figures 18-20). When the reflowed plastic material fills spaces between the individual windings of the coil 6008 and/or bonds the individual windings together, the ability of the distal end of the catheter 5700 to undergo a relatively sharp turn or bend when delivering the implant 100 implant as shown in Figures 18-20 can be impaired.

Figures 61A and 61B illustrate example implementations of a composite catheter 6100 where the plastic material is prevented from reflowing into spaces between individual windings of a portion of the coil 6008. As a result, this portion of the catheter 6100 is more flexible than an otherwise identical catheter 5700 where plastic reflowed into spaces between individual windings of the entire coil 6008. This additional flexibility can be used to enhance the ability of the distal end of the catheter 5700 to undergo a relatively sharp turn or bend when delivering the implant 100 implant as shown in Figures 18-20.

The plastic material can be prevented from reflowing into the spaces between the individual windings in a variety of different ways. For example, one or more portions of the coil 6008 can be shielded and/or one or more portions of the material that reflows can be omitted around one or more portions of the coils. Figures 61A and 61B illustrate two examples of implant catheters where plastic material is prevented from reflowing into spaces between individual windings of a portion of the coil 6008.

In the example implementation illustrated by Figure 61A, a catheter 6100 has substantially the same structure as the catheter 5700 of Figure 60, except a coil portion 6150 is shielded on one side from the material that reflows and the material that reflows is omitted on the other side of the coil portion 6150. In the example illustrated by Figure 61A, the outside of the coil portion 6150 is shielded from the material that reflows and the material that reflows is omitted on the inside of the coil portion 6150. However, in some implementations, the inside of the coil portion 6150 is shielded from the material that reflows and the material that reflows is omitted on the outside of the coil portion 6150.

The coil portion 6150 can be shielded from the material that reflows in a wide variety of different ways. For example, the outer surface of the coil portion 6150 can be wrapped, sleeved, or coated with a barrier material 6022, the inner surface of the coil portion 6150 can be sleeved, or coated with a barrier material, the wall 6010 can be wrapped, sleeved, or coated with a barrier material, and/or the liner 6112 wrapped, sleeved, or coated with a barrier material. Any manner of preventing plastic material from reflowing into the spaces between the windings of the coil 6008 can be implemented.

In the example illustrated by Figure 61A, a barrier 6022, such as a liner, tube, wrap, or coating is positioned on the outside surface of the coil portion 6150 and a gap 6020 is formed between distal and proximal portions of the wall 6010 adjacent to the inside surface of the coil portion 6150.

The barrier 6022 can take a wide variety of different forms. In one example implementation, the barrier 6022 is a tube. Such a tube can be made from a wide variety of different materials. In one example implementation, the barrier is made from a material that has a melt or flow temperature that is higher than the melt or flow temperature of the material of the jacket 5704. In one example implementation, the barrier is formed from PTFE.

In one example implementation, an adhesive is optionally used to keep this the barrier 6022 in position on the coil portion 6150. In one example implementation, the adhesive is omitted, and the reflowed material keeps the barrier in place. The ends of the barrier 6022 or the entire barrier are adhered to the coil 6008 with an adhesive, such as Polyethylene terephthalate (PET).

The gap 6020 can be formed in a variety of different ways. For example, two spaced apart sleeves can be provided or formed around the liner 5708 to form the gap or a continuous wall 6010 can be provided and then a portion can be removed to form the gap. As is mentioned above, the proximal and distal portions of the wall 6010 can be made from different materials. In one example implementation, the material of the portion of the wall on the proximal side of the gap 6020 is different than the material of the portion of the wall on the distal side of the gap. In one example implementation, the material of the portion of the wall on the proximal side of the gap 6020 is nylon and the material of the portion of the wall on the distal side of the gap is a thermoplastic elastomer, such as PBAX.

The length of the non-reflowed coil portion 6150 can be selected based on the degree of curvature, the diameter of the catheter, and the stiffness of the distal that is needed for the particular application where the catheter will be used. In some implementations, the length of the coil portion 6150 is between 50.8 mm and 254 mm (2 and 10 inches), such as between 101.6 mm and 203.2 mm (4 and 8 inches), such as between 127 mm and 177.8 mm (5 and 7 inches), such as about 152.4 mm (6 inches). Other portion 6020 lengths can be used depending on the diameter of the catheter and degree of flexibility required by the target application.

In the example implementation of the invention illustrated by Figure 61B, a catheter 6100 has substantially the same structure as the catheter 5700 of Figure 60, except a coil portion 6150 is shielded on both the inside and outside from the material that reflows. However, in some implementations, the material that reflows can be omitted on both the inside and the outside of the coil portion 6150, instead of shielding the coil portion 6150.

The coil portion 6150 can be shielded from the material that reflows in a wide variety of different ways. For example, the outer surface of the coil portion 6150 can be wrapped, sleeved, or coated with a barrier material 6022, the inner surface of the coil portion 6150 can be sleeved, or coated with a barrier material, the wall 6010 can be wrapped, sleeved, or coated with a barrier material, and/or the liner 6112 wrapped, sleeved, or coated with a barrier material. Any manner of preventing plastic material from reflowing into the spaces between the windings of the coil 6008 can be implemented.

In the example illustrated by Figure 61B, a barrier 6022, such as a liner, tube, wrap, or coating is positioned on the outside surface of the coil portion 6150 and a barrier 6109, such as a liner, tube, wrap, or coating is positioned on the inside surface of the coil portion 6150.

The barriers 6022, 6109 can take a wide variety of different forms. In one example implementation, the barriers 6022, 6109 are tubes. Such tubes can be made from a wide variety of different materials. In one example implementation, the barriers are made from a material that has a melt or flow temperature that is higher than the melt or flow temperature of the material of the jacket 5704 and the wall 6110. In one example implementation, the barriers can be formed from PTFE.

In one example implementation, an adhesive is optionally used to keep this the barriers 6022, 6109 in position on the coil portion 6150. In one implementation, the adhesive is omitted, and the reflowed material keeps the barriers in place. The ends of the barriers 6022, 6109 or the entire barrier can be adhered to the coil 6008 with an adhesive, such as Polyethylene terephthalate (PET).

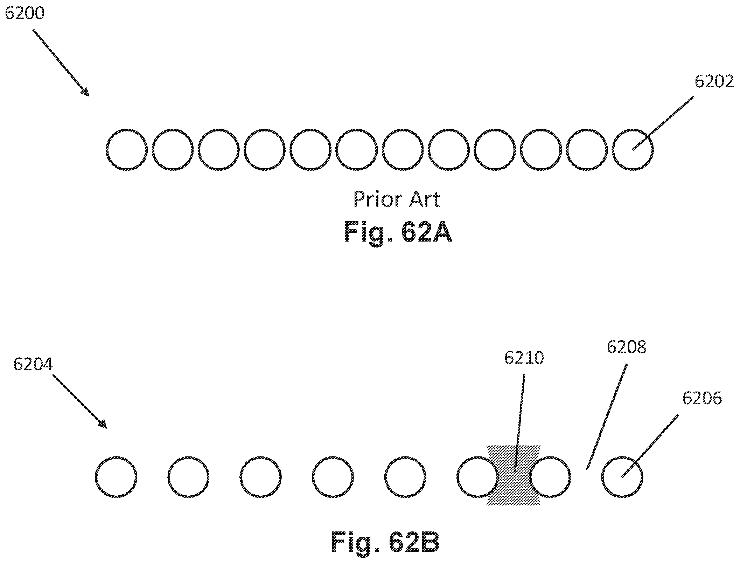

As was noted earlier herein, the coil portion of a catheter provides resistance to expansion or compression of the length of the catheter. This is important for the proper positioning and manipulation of an implant 100 or other operation performed using the catheter. Figure 62A represents a cross-section of a known coil design. As is shown, the coil 6200 is formed from a wire that is substantially round in cross-section.

Referring to Figure 62B, in one example implementation, the number of turns of wire for a given length of coil is decreased, for example, turns per inch, which increases the distance between each coil (this is sometimes referred to as pitch). Increasing the pitch increases the gap between each pair of adjacent coils. That is, the wire 6206 is wound such that a greater amount of space 6208 exists between the turns of wire 6206. This design allows for an increased level of movement between the individual coils. In order to retain a level of resistance to contraction along the length of the catheter, material 6210 such as NYLON (but can be a variety of materials, including any of the materials described previously with respect to catheter layers herein) is located adjacent to the coil 6204 and is softened with heat or other methods so as to flow into the space between the individual wires 6206. The result is a coil that is more flexible than known designs 6200 but still resistant to compression.

Figure 62B illustrates material 6210 after it has flowed between two turns of the coil. The catheter can be customized to have the material 6210 flow between all the turns of the coil, some of the turns of the coil, or one pair of turns. This can be customized to accomplish different characteristics of the final implant catheter. Material 6210 between each turn will have the strongest resistance to compression, but material between various turns can be omitted for more flexibility and/or some additional compression. In one implementation, the material 6210 flows between every other pair of turns. In one implementation, the material 6210 flows between all the turns of the coil. In one implementation, the material 6210 flows between 70-95% of the turns of the coil.

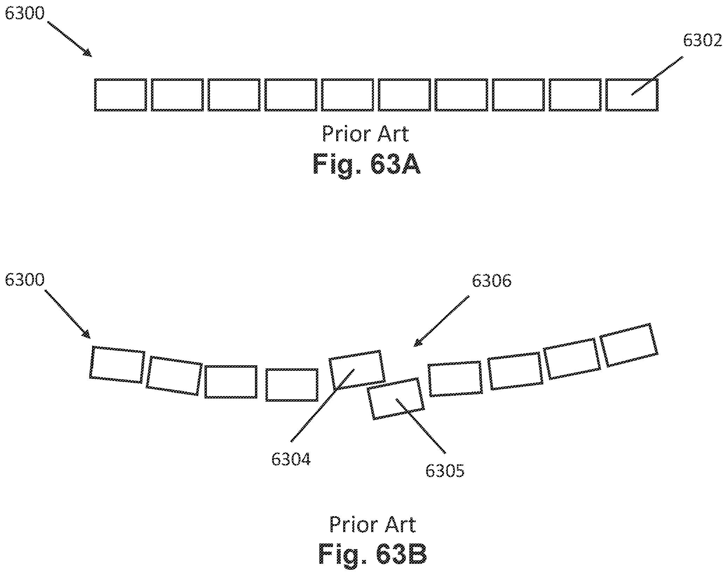

A rectangular cross-section can be used to further enhance a catheter's resistance to compression and elongation. Such a coil 6300 is illustrated in Figures 63A and 63B. A coil with a rectangular shape of the wire 6302 can provides a flat surface at each wire 6302 which aligns with the next winding of the coil to prevent compression of the coil. However, as illustrated in Figure 63B, when the coil 6300 is caused to bend, the coil wires (for example, 6304 and 6305) can become misaligned and slip past each other as illustrated at 6306. The result is a catheter that has a reduced ability to resist compressive forces when it is bent. Additionally, slipping coils can cause distortion of the inner and outer surfaces of a catheter.

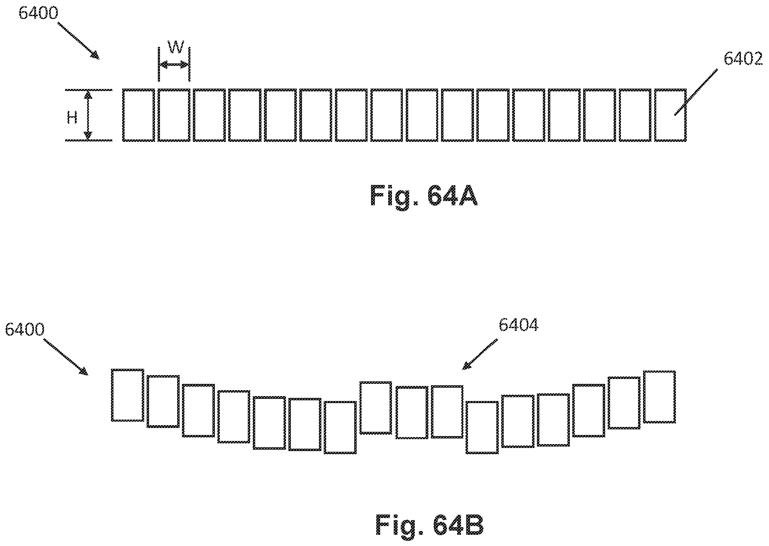

Figures 64A and 64B illustrate an improved example of a rectangular cross section coil 6400. In this example, the wire 6402 has a height H that is larger than its width W. This configuration reduces the chance that the wires will become misaligned. For example, as is shown in the coil of Figure 64B, the coils do not slip past each other.

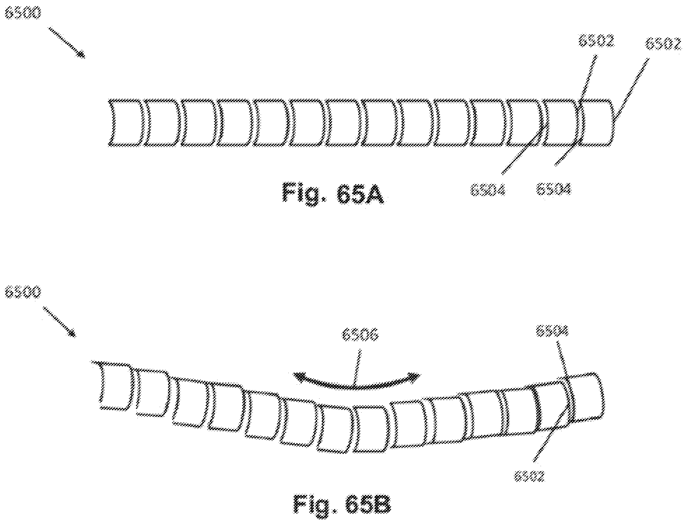

In an example implementation, a coil is formed with an "nose" and "socket" arrangement. Such a coil 6500 is illustrated in Figures 65A and 65B. As is visible in in Figure 65A, the wire used to form the coil has a convex face 6502 and a concave face 6504. When the wire is formed into a coil 6500, the wire is wound in an orientation wherein the convex face 6502 is oriented such that it faces a concave face 6504. The result is that the concave face 6504 acts to hold the convex face 6502 in alignment with the concave face 6504. As is illustrated in Figure 65B, as the coil is flexed or bent 6506, the convex face 6502 intrudes into the space formed by the concave face 6504 of the adjacent wire. The result is that the individual wires are prevented from slipping out of place as was illustrated for the wire coils shown in Figure 63B.

While various inventive aspects, concepts and features of the disclosures may be described and illustrated herein as embodied in combination in the example embodiments, these various aspects, concepts, and features may be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures-such as alternative materials, structures, configurations, methods, devices, and components, alternatives as to form, fit, and function, and so on-may be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein.

Additionally, even though some features, concepts, or aspects of the disclosures may be described herein as being a preferred arrangement or method, such description is not intended to suggest that such feature is required or necessary unless expressly so stated. Still further, example or representative values and ranges may be included to assist in understanding the present application, however, such values and ranges are not to be construed in a limiting sense and are intended to be critical values or ranges only if so expressly stated.

Moreover, while various aspects, features and concepts may be expressly identified herein as being inventive or forming part of a disclosure, such identification is not intended to be exclusive, but rather there may be inventive aspects, concepts, and features that are fully described herein without being expressly identified as such or as part of a specific disclosure, the disclosures instead being set forth in the appended claims. Descriptions of example methods or processes are not limited to inclusion of all steps as being required in all cases, nor is the order that the steps are presented to be construed as required or necessary unless expressly so stated. Further, the techniques, methods, operations, steps, etc. described or suggested herein can be performed on a living animal or on a non-living simulation, such as on a cadaver, cadaver heart, simulator (e.g. with the body parts, tissue, etc. being simulated), etc. The words used in the claims have their full ordinary meanings and are not limited in any way by the description of the embodiments in the specification.

## Claims

1. An implant catheter for heart valve repair which comprises:
an outer jacket (5704);
one or more layers (5708, 6006, 6010) positioned inside the outer jacket (5704);
a reinforcement layer (6008) positioned inside the outer jacket (5704); and
a first leakage shield (6022) located adjacent to the reinforcement layer (6008) and situated between the reinforcement layer (6008) and the outer jacket (5704);
wherein at least one of the outer jacket (5704) and the one or more of the layers are reflowed to bond the jacket, the reinforcement layer, and the one or more layers (5708, 6006, 6010) together;
wherein a portion (6150) of the reinforcement layer (6008) is free from all material of the outer jacket (5704) and the one or more layers (5708, 6006, 6010) that are reflowed; and
wherein the first leakage shield (6022) prevents material of the outer jacket (5704) and the one or more layers (5708, 6006, 6010) that are reflowed from contacting the portion (6150) of the reinforcement layer (6008).

2. The implant catheter of claim 1, further comprising a plurality of sub-lumens (5712).

3. The implant catheter of any one of claims 1 or 2, wherein a first portion of the outer jacket (5704) is formed from a material with an enhanced level of flexibility with regard to the remaining portions of the outer jacket (5704).

4. The implant catheter of any one of claims 1 to 3, wherein the reinforcement layer is a coil layer (6008) formed from self-aligning wire.

5. The implant catheter of any one of claims 1 to 4
wherein a portion (6020) of at least one of the plurality of layers is omitted along a section of the implant catheter; and
the first leakage shield (6022) is located adjacent to the reinforcement layer (6008) in the area of the omitted layer portion (6020).

6. The implant catheter of claim 5, wherein the first leakage shield (6022) is formed from polytetrafluoroethylene.

7. The implant catheter of any one of claims 1 to 6 further comprising a main lumen (5710); wherein:
the plurality of layers are positioned between the outer jacket (5704) and the main lumen (5710); and
a second leakage shield (6109) is located adjacent to the reinforcement layer (6008) and situated between the reinforcement layer (6008) and main lumen (5710).

8. The implant catheter of any one of claims 1 to 7, wherein the reinforcement layer (6008) is at least one of a coil, a coil layer, a spring, a spring layer, a braid, and a braided layer.

9. A method of manufacturing an implant catheter for heart valve repair comprising:
arranging a plurality of tubular layers (5708, 6006, 6010) and a reinforcement layer (6008) radially inside an outer jacket (5704);
reflowing at least one of the outer jacket (5704) and one or more of the plurality of layers (5708, 6006, 6010) to bond the outer jacket (5704), the reinforcement layer (6008), and the plurality of layers together (5708, 6006, 6010); and
positioning a first leakage shield (6022) adjacent to the reinforcement layer (6008) between the reinforcement layer (6008) and the outer jacket (5704) prior to reflowing.

10. The method of claim 9, wherein the reinforcement layer (6008) is at least one of a coil, a coil layer, a spring, a spring layer, a braid, and a braided layer.

11. The method of claim 9, further comprising positioning a second leakage shield (6109) located adjacent to the reinforcement layer (6008) and radially inside the reinforcement layer (6008).

12. The implant catheter of any one of claims 1 to 8 wherein:
the reinforcement layer is a coil layer;
the coil layer (6008) includes gaps (6208) between one or more pairs of adjacent coils (6400; 6500) of the coil layer (6008); and
a polymeric material (6210) is disposed in one or more of the gaps (6208).

13. The implant catheter of claim 12 wherein the polymeric material (6210) comprises nylon.

14. The implant catheter of any one of claims 12 to 13 wherein the polymeric material (6210) is disposed in all of the gaps (6208).

15. The implant catheter of any one of claims 12 to 13 wherein the polymeric material (6210) is disposed in some of the gaps (6208); preferably wherein the polymeric material (6210) is disposed between 70-95% of the turns of the coil.

## Patentansprüche

1. Implantationskatheter zur Reparatur von Herzklappen, umfassend:
einen Außenmantel (5704);
eine oder mehrere Schichten (5708, 6006, 6010), die innerhalb des Außenmantels (5704) angeordnet sind;
eine Verstärkungsschicht (6008), die innerhalb des Außenmantels (5704) angeordnet ist; und
eine erste Leckageabschirmung (6022), die benachbart zu der Verstärkungsschicht (6008) und zwischen der Verstärkungsschicht (6008) und dem Außenmantel (5704) angeordnet ist;
wobei wenigstens eines von dem Außenmantel (5704) und der einen oder den mehreren Schichten aufgeschmolzen wird, um den Mantel, die Verstärkungsschicht und die eine oder die mehreren Schichten (5708, 6006, 6010) miteinander zu verbinden;
wobei ein Abschnitt (6150) der Verstärkungsschicht (6008) frei von sämtlichem Material des Außenmantels (5704) und der einen oder der mehreren Schichten (5708, 6006, 6010), die aufgeschmolzen werden, ist; und
wobei die erste Leckageabschirmung (6022) verhindert, dass Material des Außenmantels (5704) und der einen oder der mehreren Schichten (5708, 6006, 6010), die aufgeschmolzen werden, mit dem Abschnitt (6150) der Verstärkungsschicht (6008) in Kontakt ist.

2. Implantationskatheter nach Anspruch 1, der ferner mehrere Nebenlumina (5712) umfasst.

3. Implantationskatheter nach einem der Ansprüche 1 oder 2, wobei ein erster Abschnitt des Außenmantels (5704) aus einem Material gebildet ist, das in Bezug auf die übrigen Abschnitte des Außenmantels (5704) einen erhöhten Grad an Flexibilität aufweist.

4. Implantationskatheter nach einem der Ansprüche 1 bis 3, wobei die Verstärkungsschicht eine Wendelschicht (6008) ist, die aus selbstausrichtendem Draht gebildet ist.

5. Implantationskatheter nach einem der Ansprüche 1 bis 4,
wobei ein Abschnitt (6020) von wenigstens einer der mehreren Schichten entlang eines Abschnitts des Implantationskatheters weggelassen ist; und
die erste Leckageabschirmung (6022) benachbart zu der Verstärkungsschicht (6008) in dem Bereich des weggelassenen Schichtabschnitts (6020) angeordnet ist.

6. Implantationskatheter nach Anspruch 5, wobei die erste Leckageabschirmung (6022) aus Polytetrafluorethylen gebildet ist.

7. Implantationskatheter nach einem der Ansprüche 1 bis 6, der ferner ein Hauptlumen (5710) umfasst; wobei:
die mehreren Schichten zwischen dem Außenmantel (5704) und dem Hauptlumen (5710) angeordnet sind; und
eine zweite Leckageabschirmung (6109) benachbart zu der Verstärkungsschicht (6008) und zwischen der Verstärkungsschicht (6008) und dem Hauptlumen (5710) angeordnet ist.

8. Implantationskatheter nach einem der Ansprüche 1 bis 7, wobei die Verstärkungsschicht (6008) wenigstens eines von einer Wendel, einer Wendelschicht, einer Feder, einer Federschicht, einem Geflecht und einer Geflechtschicht ist.

9. Verfahren zum Herstellen eines Implantationskatheters zur Reparatur von Herzklappen, umfassend:
Anordnen mehrerer röhrenförmiger Schichten (5708, 6006, 6010) und einer Verstärkungsschicht (6008) radial innerhalb eines Außenmantels (5704);
Aufschmelzen von wenigstens einem von dem Außenmantel (5704) und einer oder mehreren der mehreren Schichten (5708, 6006, 6010), um den Außenmantel (5704), die Verstärkungsschicht (6008) und die mehreren Schichten (5708, 6006, 6010) miteinander zu verbinden; und
Positionieren einer ersten Leckageabschirmung (6022) benachbart zu der Verstärkungsschicht (6008) zwischen der Verstärkungsschicht (6008) und dem Außenmantel (5704) vor dem Aufschmelzen.

10. Verfahren nach Anspruch 9, wobei die Verstärkungsschicht (6008) wenigstens eines von einer Wendel, einer Wendelschicht, einer Feder, einer Federschicht, einem Geflecht und einer Geflechtschicht ist.

11. Verfahren nach Anspruch 9, das ferner das Positionieren einer zweiten Leckageabschirmung (6109) umfasst, die benachbart zu der Verstärkungsschicht (6008) und radial innerhalb der Verstärkungsschicht (6008) angeordnet ist.

12. Implantationskatheter nach einem der Ansprüche 1 bis 8, wobei:
die Verstärkungsschicht eine Wendelschicht ist;
die Wendelschicht (6008) Lücken (6208) zwischen einem oder mehreren Paaren von benachbarten Windungen (6400; 6500) der Wendelschicht (6008) aufweist; und
ein Polymermaterial (6210) in einer oder mehreren der Lücken (6208) angeordnet ist.

13. Implantationskatheter nach Anspruch 12, wobei das Polymermaterial (6210) Nylon umfasst.

14. Implantationskatheter nach einem der Ansprüche 12 bis 13, wobei das Polymermaterial (6210) in allen Lücken (6208) angeordnet ist.

15. Implantationskatheter nach einem der Ansprüche 12 bis 13, wobei das Polymermaterial (6210) in einigen der Lücken (6208) angeordnet ist, wobei vorzugsweise das Polymermaterial (6210) zwischen 70-95% der Windungen der Wendel angeordnet ist.

## Revendications

1. Cathéter d'implant pour la réparation d'une valvule cardiaque qui comprend :
une gaine externe (5704);
une ou plusieurs couches (5708, 6006, 6010) positionnées à l'intérieur de la gaine externe (5704);
une couche de renforcement (6008) positionnée à l'intérieur de la gaine externe (5704); et
un premier écran de fuite (6022) situé de manière adjacente à la couche de renforcement (6008) et situé entre la couche de renforcement (6008) et la gaine externe (5704);
au moins un élément parmi la gaine externe (5704) et ladite une ou lesdites plusieurs couches étant refondu pour lier la gaine, la couche de renforcement et ladite une ou lesdites plusieurs couches (5708, 6006, 6010) ensemble;
une partie (6150) de la couche de renforcement (6008) étant exempte de tout matériau de la gaine externe (5704) et de ladite une ou desdites plusieurs couches (5708, 6006, 6010) qui sont refondues; et
le premier écran de fuite (6022) empêchant le matériau de la gaine externe (5704) et de ladite une ou desdites plusieurs couches (5708, 6006, 6010) qui sont refondues d'entrer en contact avec la partie (6150) de la couche de renforcement (6008).

2. Cathéter d'implant selon la revendication 1, comprenant en outre une pluralité de sous-lumières (5712).

3. Cathéter d'implant selon l'une quelconque des revendications 1 ou 2, une première partie de la gaine externe (5704) étant formée à partir d'un matériau présentant un niveau de flexibilité augmenté par rapport aux parties restantes de la gaine externe (5704).

4. Cathéter d'implant selon l'une quelconque des revendications 1 à 3, la couche de renforcement étant une couche de bobines (6008) formée à partir d'un fil à autoalignement.

5. Cathéter d'implant selon l'une quelconque des revendications 1 à 4
une partie (6020) d'au moins l'une parmi la pluralité de couches étant omise le long d'une section du cathéter d'implant; et
le premier écran de fuite (6022) étant situé de manière adjacente à la couche de renforcement (6008) dans la zone de la partie (6020) de couche omise.

6. Cathéter d'implant selon la revendication 5, le premier écran de fuite (6022) étant formé à partir de polytétrafluoroéthylène.

7. Cathéter d'implant selon l'une quelconque des revendications 1 à 6, comprenant en outre une lumière principale (5710);
la pluralité de couches étant positionnées entre la gaine externe (5704) et la lumière principale (5710); et
un second écran de fuite (6109) étant situé de manière adjacente à la couche de renforcement (6008) et situé entre la couche de renforcement (6008) et la lumière principale (5710).

8. Cathéter d'implant selon l'une quelconque des revendications 1 à 7, la couche de renforcement (6008) étant au moins un élément parmi une bobine, une couche de bobines, un ressort, une couche de ressorts, une tresse et une couche tressée.

9. Procédé de fabrication d'un cathéter d'implant pour la réparation d'une valvule cardiaque comprenant :
l'agencement d'une pluralité de couches tubulaires (5708, 6006, 6010) et d'une couche de renforcement (6008) radialement à l'intérieur d'une gaine externe (5704);
la refonte d'au moins un élément parmi la gaine externe (5704) et une ou plusieurs couches parmi la pluralité de couches (5708, 6006, 6010) pour lier la gaine externe (5704), la couche de renforcement (6008) et la pluralité de couches ensemble (5708, 6006, 6010); et
le positionnement d'un premier écran de fuite (6022) de manière adjacente à la couche de renforcement (6008) entre la couche de renforcement (6008) et la gaine externe (5704) avant la refonte.

10. Procédé selon la revendication 9, la couche de renforcement (6008) étant au moins un élément parmi une bobine, une couche de bobines, un ressort, une couche de ressorts, une tresse et une couche tressée.

11. Procédé selon la revendication 9, comprenant en outre le positionnement d'un second écran de fuite (6109) situé de manière adjacente à la couche de renforcement (6008) et radialement à l'intérieur de la couche de renforcement (6008).

12. Cathéter d'implant selon l'une quelconque des revendications 1 à 8 :
la couche de renforcement étant une couche de bobines;
la couche de bobines (6008) comprenant des interstices (6208) entre une ou plusieurs paires de bobines (6400; 6500) adjacentes de la couche de bobines (6008); et
un matériau polymère (6210) étant disposé dans un ou plusieurs des interstices (6208).

13. Cathéter d'implant selon la revendication 12, le matériau polymère (6210) comprenant du nylon.

14. Cathéter d'implant selon l'une quelconque des revendications 12 à 13, le matériau polymère (6210) étant disposé dans tous les interstices (6208).

15. Cathéter d'implant selon l'une quelconque des revendications 12 à 13, le matériau polymère (6210) étant disposé dans certains interstices (6208); de préférence, le matériau polymère (6210) étant disposé entre 70 et 95 % des spires de la bobine.
